(19) Europäisches Patentamt
European Patent Office
Office européen des brevets

(11) Veröffentlichungsnummer: **0 298 921 B1**

(12) **EUROPÄISCHE PATENTSCHRIFT**

(45) Veröffentlichungstag der Patentschrift: **23.12.92**

(51) Int. Cl.[5]: **C07D 413/06**, C07D 417/06, C07D 471/04

(21) Anmeldenummer: **88810457.7**

(22) Anmeldetag: **04.07.88**

Die Akte enthält technische Angaben, die nach dem Eingang der Anmeldung eingereicht wurden und die nicht in dieser Patentschrift enthalten sind.

(54) **1,2-Benzisoxazole und 1,2-Benzisothiazole.**

(30) Priorität: **08.07.87 US 71124**

(43) Veröffentlichungstag der Anmeldung:
**11.01.89 Patentblatt 89/02**

(45) Bekanntmachung des Hinweises auf die Patenterteilung:
**23.12.92 Patentblatt 92/52**

(84) Benannte Vertragsstaaten:
**AT BE CH DE ES FR GB GR IT LI LU NL SE**

(56) Entgegenhaltungen:
**EP-A- 0 192 060**
**US-A- 4 723 021**

(73) Patentinhaber: **CIBA-GEIGY AG**
**Klybeckstrasse 141**
**CH-4002 Basel(CH)**

(72) Erfinder: **Bowman, Robert M.**
**6 Meadowbrook Court**
**Summit, New Jersey 07901(US)**

EP 0 298 921 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung sind 1,2-Benzisoxazole und 1,2-Benzisothiazole der Formel I

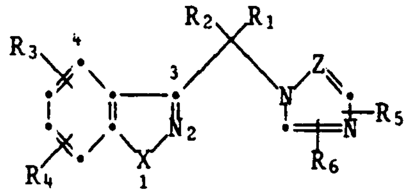

(I)

worin X Sauerstoff oder Schwefel, Z die Gruppe -CH= zur Bildung der Imidazol-1-yl-Gruppe oder ein Stickstoffheteroatom zur Bildung der 1,2,4-Triazol-1-yl-Gruppe, $R_1$ und $R_2$ unabhängig voneinander Wasserstoff, Niederalkyl, Niederalkenyl, Niederalkinyl oder Arylniederalkyl oder $R_1$ und $R_2$ zusammen Niederalkylen oder $R_1$ zusammen mit $R_5$ als Substituent des C-Atoms Z in der Imidazolylgruppe $C_2$-$C_4$-Alkylen, $R_3$ und $R_4$ unabhängig voneinander Wasserstoff, Niederalkyl, Cycloalkyl, Halogen, Trifluormethyl, Cyan, Nitro, Amino, Hydroxy, Niederalkanoyloxy, carbocyclisches Aroyloxy, Niederalkoxy oder carbocyclisches Aryl, $R_3$ und $R_4$ zusammen an nachbarständigen C-Atomen Niederalkylendioxy, $R_3$ und $R_4$ zusammen an nachbarständigen C-Atomen 1,3-Butadienylen oder $C_3$-$C_5$-Alkylen, wobei $R_3$ und $R_4$ mit diesen nachbarständigen C-Atomen eine entsprechende benzokondensierte Gruppe oder eine kondensierte $C_5$-$C_7$-Cycloalkenylgruppe ergibt, $R_5$ an einem Ring-C-Atom Wasserstoff, Niederalkyl oder Hydroxyniederalkyl, $R_6$ an einem Ring-C-Atom Wasserstoff oder Niederalkyl oder $R_5$ und $R_6$ zusammen $C_3$-$C_5$-Alkylen darstellen und dabei Z ein C-Atom darstellt und $R_5$ und $R_6$ zusammen dieses C-Atom und das nachbarständige C-Atom substituieren, und Salze dieser Verbindungen.

Die mit dem Ausdruck „nieder" definierten Substituenten besitzen bis zu 7 C-Atome

Die vorliegende Erfindung betrifft ferner Verfahren zur Herstellung von Verbindungen der Formel I, pharmazeutische Präparate mit Verbindungen der Formel I und ein Verfahren zur Behandlung von Zuständen und Krankheiten, welche sich durch Konvulsionen äussern, und zwar durch Applikation einer Verbindung der Formel I bzw. einer pharmazeutischen Zusammensetzung enthaltend eine Verbindung der Formel I.

Die Verbindungen der Formel I sind besonders wertwolle Antikonsulsiva mit breitem Wirkungsspektrum. Diese Verbindungen können allein oder in Kombination mit anderen Verbindungen zur Behandlung von Störungen und Krankheiten, die mit Konvulsionen verlaufen, z.B. Epilepsien wie Grand Mal und Petit Mal, sowie generell epileptischen Zuständen verwendet werden.

Besonders bevorzugt sind Verbindung der Formel I und deren pharmazeutisch annehmbaren Salze, worin

a) X Sauerstoff und Z die Gruppen -CH= zur Bildung der Imidazol-1-yl-Gruppe,

b) X Sauerstoff und Z ein Stickstoffheteroatom zur Bildung der 1,2,4-Triazol-1-yl-Gruppe,

c) X Schwefel und Z die Gruppe -CH= und

d) X Schwefel und Z ein Stickstoffheteroatom bedeuten.

Bevorzugt sind Verbindungen der Formel I und deren pharmazeutisch annehmbaren Salze, worin

a) $R_1$ und $R_2$ unabhängig voneinander Wasserstoff, Niederalkyl, Niederalkenyl, Niederalkinyl oder Arylniederalkyl oder zusammen Niederalkylen bedeuten, und

b) Z die Gruppe -CH= zur Bildung der Imidazol-1-yl-Gruppe, $R_1$ zusammen mit $R_5$ als Substituent des C-Atoms der Imidazolyl-Gruppe $C_2$–$C_4$-Alkylen und $R_2$ Wasserstoff oder Niederalkyl bedeuten.

Ebenfalls bevorzugt sind Verbindungen der Formel I und deren Salze, worin

a) $R_3$ und $R_4$ unabhängig voneinander Wasserstoff, Niederalkyl, Cycloalkyl, Halogen, Trifluoromethyl, Cyan, Nitro, Amino, Hydroxy, Niederalkanoyloxy, carbocyclisches Aroyloxy, Niederalkoxy oder carbocyclisches Aryl oder $R_3$ und $R_4$ zusammen an nachbarständigen C-Atomen Niederalkylendioxy oder

b) $R_3$ und $R_4$ zusammen an nachbarständigen C-Atomen 1,3-Butadienylen, wobei $R_3$ und $R_4$ mit diesen nachbarständigen C-Atomen eine entsprechende benzokondensierte Gruppe ergibt, oder

c) $R_3$ und $R_4$ zusammen an nachbarständigen C-Atomen $C_3$-$C_5$-Alkylen bedeuten, wobei $R_3$ und $R_4$ mit diesen nachbarständigen C-Atomen eine kondensierte $C_5$-$C_7$-Alkenylgruppe ergibt.

Bevorzugter Erfindungsgegenstand sind Verbindungen der Formel I und pharmazeutisch annehmbare Salze davon, worin X Sauerstoff und Z die Gruppe -CH= bedeuten, insbesondere Imidazolylmethyl-substituierte 1,2-Benzisoxazol-Derivate der Formel IIa

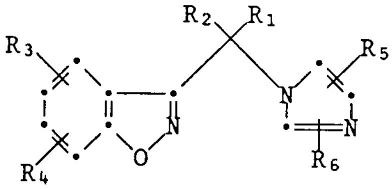

(IIa)

worin $R_1$ und $R_2$ unabhängig voneinander Wasserstoff, Niederalkyl, Arylniederalkyl, Niederalkenyl oder Niederalkinyl oder $R_1$ und $R_2$ zusammen Niederalkylen, $R_3$ und $R_4$ unabhängig voneinander Wasserstoff, Niederalkyl, Cycloalkyl, Halogen, Trifluormethyl, Cyan, Nitro, Amino, Hydroxy, Niederalkanoyloxy, carbocyclisches Aroyloxy, Niederalkoxy oder carbocyclisches Aryl oder $R_3$ und $R_4$ zusammen an nachbarständigen C-Atomen Niederalkylendioxy und $R_5$ und $R_6$ unabhängig voneinander Wasserstoff oder Niederalkyl bedeuten, und pharmazeutisch annehmbare Salze dieser Verbindungen.

Bevorzugt sind Verbindungen der Formel IIa, worin $R_1$ Wasserstoff oder Niederalkyl, $R_2$ Wasserstoff oder Niederalkyl, $R_3$ Wasserstoff, Niederalkyl, $C_5$-$C_7$-Cycloalkyl, Halogen, Trifluormethyl, Cyan, Nitro, Amino, Hydroxy, Niederalkanoyloxy, carbocyclisches Aroyloxy, Niederalkoxy oder carbocyclisches Aryl, $R_4$ Wasserstoff, Niederalkyl, Niederalkoxy oder Halogen oder $R_3$ und $R_4$ zusammen an nachbarständigen C-Atomen Niederalkylendioxy und $R_5$ und $R_6$ unabhänging voneinander Wasserstoff oder Niederalkyl bedeuten, und pharmazeutisch annehmbare Salze davon.

Ebenfalls bevorzugt sind Verbindungen der Formel IIa, worin $R_1$ Wasserstoff oder Niederalkyl, $R_2$ Wasserstoff oder Niederalkyl, $R_3$ Wasserstoff, Niederalkyl, Halogen, Trifluormethyl oder Phenyl, $R_4$ Wasserstoff, Niederalkyl oder Halogen bedeuten, $R_5$ sich in 4-Stellung des Imidazolylrings befindet und Wasserstoff oder Niederalkyl bedeutet, $R_6$ sich in 5-Stellung des Imidazolylrings befindet und Wasserstoff oder Niederalkyl bedeutet, und pharmazeutisch annehmbare Salze davon.

Besonders bevorzugt sind Verbindungen der Formel IIa, worin $R_1$ Wasserstoff oder Niederalkyl, $R_2$ Wasserstoff, $R_3$ Wasserstoff, Niederalkyl, Trifluormethyl, Halogen oder Phenyl und $R_4$, $R_5$ und $R_6$ Wasserstoff bedeuten, und pharmazeutisch annehmbare Salze davon.

Ein weiterer bevorzugter Erfindungsgegenstand sind Triazolylmethyl-substituierte 1,2-Benzisoxazol-Derivate der Formel IIb

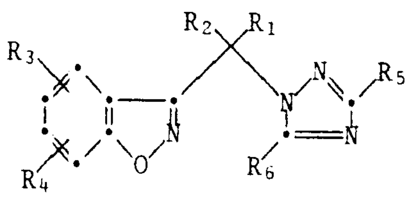

(IIb)

worin $R_1$ und $R_2$ unabhängig voneinander Wasserstoff, Niederalkyl, Arylniederalkyl, Niederalkenyl oder Niederalkinyl oder $R_1$ und $R_2$ zusammen Niederalkylen, $R_3$ und $R_4$ unabhängig voneinander Wasserstoff, Niederalkyl, Cycloalkyl, Halogen, Trifluormethyl, Cyan, Nitro, Amino, Hydroxy, Niederalkanoyloxy, carbocyclisches Aroyloxy, Niederalkoxy oder carbocyclisches Aryl oder $R_3$ und $R_4$ zusammen an nachbarständigen C-Atomen Niederalkylendioxy und $R_5$ und $R_6$ unabhängig voneinander Wasserstoff oder Niederalkyl bedeuten, und pharmazeutisch annehmbare Salze dieser Verbindungen.

Bevorzugt sind Verbindungen der Formel IIb, worin $R_1$ Wasserstoff oder Niederalkyl, $R_2$ Wasserstoff oder Niederalkyl, $R_3$ Wasserstoff, Niederalkyl, $C_5$-$C_7$-Cycloalkyl, Halogen, Trifluormethyl, Cyan, Nitro, Amino, Hydroxy, Niederalkanoyloxy, carbocyclisches Aroyloxy, Niederalkoxy oder carbocyclisches Aryl, $R_4$ Wasserstoff, Niederalkyl, Niederalkoxy oder Halogen oder $R_3$ und $R_4$ zusammen an nachbarständigen C-Atomen Niederalkylendioxy oder $R_3$ und $R_4$ zusammen an nachbarständigen C-Atomen 1,3-Butadienylen oder $C_3$-$C_5$-Alkylen, wobei $R_3$ und $R_4$ mit diesen nachbarständigen C-Atomen eine entsprechende benzokondensierte Gruppe oder eine kondensierte $C_5$-$C_7$-Cycloalkenylgruppe ergibt, und $R_5$ und $R_5$ unabhängig voneinander Wasserstoff oder Niederalkyl bedeuten, und pharmazeutisch annehmbare Salze dieser Verbindungen.

Ebenfalls bevorzugt sind Verbindungen der Formel IIb, worin $R_1$ Wasserstoff oder Niederalkyl, $R_2$ Wasserstoff oder Niederalkyl, $R_3$ Wasserstoff, Niederalkyl, Halogen, Trifluormethyl oder Phenyl, $R_4$ Wasserstoff, Niederalkyl oder Halogen und $R_5$ und $R_6$ Wasserstoff oder Niederalkyl bedeuten, und pharmazeutisch annehmbare Salze dieser Verbindungen.

Besonders bevorzugt sind Verbindungen der Formel IIb, worin $R_1$ Wasserstoff oder Niederalkyl, $R_2$ Wasserstoff, $R_3$ Wasserstoff, Niederalkyl, Trifluormethyl, Halogen oder Phenyl und $R_4$, $R_5$ und $R_6$ Wasserstoff bedeuten, und pharmazeutisch annehmbare Salze davon.

Bevorzugte Erfindungsgegenstände sind ausserdem 1,2-Benzisothiazol-Derivate, welche den genannten 1,2-Benzisoxazolderivaten der Formeln IIa und IIb entsprechen, indem der Sauerstoff in der Benzisoxazol-Gruppe durch Schwefel ersetzt wurde. In diesen Verbindungen sind die für die 1,2-Benzisoxalderivate bevorzugt genannten Gruppen $R_1$-$R_5$ ebenfalls bevorzugt.

Bevorzugter Erfindungsgegenstand sind ferner Naphth[2,3-d]-(isoxazol oder isothiazol)-Derivate der Formel IIIa und Naphth[2,1-d]-(isoxazol oder isothiazol)-Derivate der Formel IIIb:

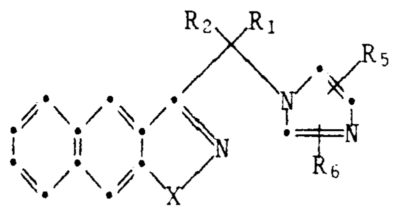

(IIIa)

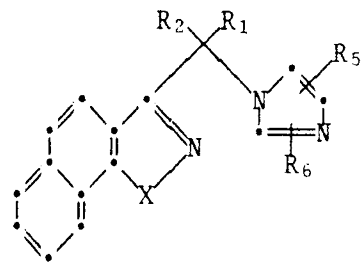

(IIIb)

worin X Sauerstoff oder Schwefel, $R_1$, $R_2$, $R_5$ und $R_6$ unabhängig voneinander Wasserstoff oder Niederalkyl bedeuten, und pharmazeutisch annehmbare Salze davon.

Bevorzugt sind dabei Verbindungen der Formeln IIIa und IIIb, worin X Sauerstoff bedeutet.

Ein weiterer Erfindungsgegenstand bezieht sich auf Verbindungen der Formel IIIc:

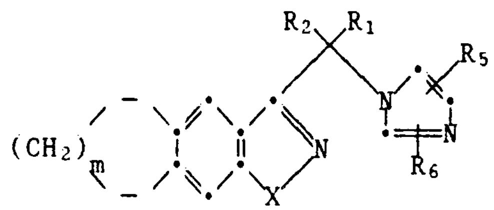

(IIIc),

worin X Sauerstoff oder Schwefel, m die Zahlen drei, vier oder fünf, insbesondere die Zahlen drei oder vier, $R_1$, $R_2$, $R_5$ und $R_6$ unabhängig voneinander Wasserstoff oder Niederalkyl bedeuten, und pharmazeutisch annehmbare Salze dieser Verbindungen.

Bevorzugt sind Verbindungen der Formel IIIc, worin X Sauerstoff und m die Zahlen 3 und 4 bedeutet.

Ein weiterer bevorzugter Erfindungsgegenstand sind Verbindungen der Formel IV:

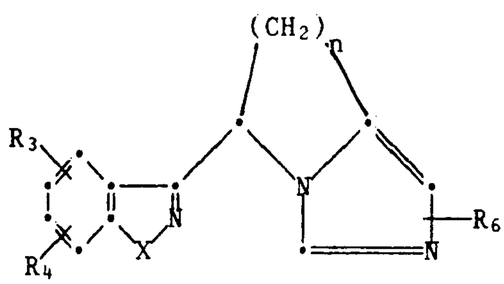

(IV)

worin n die Zahlen zwei, drei oder vier, X Sauerstoff oder Schwefel, $R_3$ Wasserstoff, Niederalkyl, Trifluormethyl, Halogen oder Phenyl, $R_4$ Wasserstoff, Niederalkyl oder Halogen und $R_6$ Wasserstoff oder Niederalkyl bedeuten, und pharmazeutisch annehmbare Salze dieser Verbindungen.

Ebenfalls bevorzugt sind Verbindungen der Formel IV, worin $R_3$ Wasserstoff, Niederalkyl, Trifluormethyl oder Phenyl und $R_4$ und $R_6$ Wasserstoff bedeuten, und pharmazeutisch annehmbare Salze dieser Verbindungen.

Besonders bevorzugt sind solche Verbindungen der Formel IV, worin X Sauerstoff bedeutet und n zwei oder drei ist, und pharmazeutisch annehmbare Salze dieser Verbindungen.

Insbesondere sind solche Verbindungen der Formel IV bevorzugt, worin X Sauerstoff, n die Zahl drei, $R_3$ Wasserstoff, Niederalkyl, Halogen, Trifluormethyl oder Phenyl und $R_4$ und $R_6$ Wasserstoff bedeuten, und pharmazeutisch annehmbare Salze dieser Verbindungen.

Die weiter vorn und im folgenden verwendeten Bezeichnungen und Definitionen haben im Rahmen der Beschreibung der vorliegenden Erfindung folgende Bedeutungen:
Der im Zusammenhang mit Gruppen, Substituenten oder Verbindungen verwendete Ausdruck "nieder" bedeutet, dass diese bis zu sieben, vorzugsweise bis zu vier C-Atome enthalten.

Niederalkyl ist vorzugsweise geradkettig aber auch verzweigt mit vorzugsweise ein bis vier C-Atomen, z.B. Ethyl, Propyl, Isopropyl, n-Butyl, tert-Butyl oder Isobutyl, insbesondere Methyl.

Niederalkenyl hat vorzugsweise 3 bis 7-C-Atome und bedeutet z.B. Allyl.

Niederalkinyl hat vorzugsweise 3 bis 7 C-Atome und bedeutet z.B. Propargyl.

Eine Niederalkylenbrücke hat vorzugsweise 1-4 C-Atome und ist z.B. Methylen, Ethylen, Propylen, z.B. 1,2- oder 1,3-Propylen, oder Butylen, z.B. 1,2-, 1,3- oder 1,4-Butylen.

Niederalkylendioxy ist vorzugsweise Methylendioxy oder Ethylendioxy.

Niederalkoxy hat vorzugsweise 1-4 C-Atome, z.B. Ethoxy, Propoxy oder vorzugsweise Methoxy.

Niederalkanoyl hat vorzugsweise 2-4 C-Atome und ist z.B. Acetyl, Propionyl, n-Butyryl, Isobutyryl oder Pivaloyl.

Niederalkanoyloxy ist insbesondere Acetoxy, Propionyloxy, n- oder Isobutyryloxy oder Pivaloyloxy.

Halogen ist vorzugsweise Fluor, Chlor oder Brom, ferner Iod.

Carbocyclisches Aroyl ist vorzugsweise Benzoyl oder Benzoyl mit ein bis drei Substituenten der Gruppe Niederalkyl, Niederalkoxy, Trifluormethyl und Halogen, ferner 1- oder 2-Naphthoyl.

Carbocyclisches Aroyloxy ist vorzugsweise Benzoyloxy oder Benzoyloxy substituiert durch Trifluormethyl, Niederalkyl, Halogen oder Niederalkoxy, insbesondere durch Methyl, Chlor oder Methoxy, oder ist 1- oder 2-Naphthoyloxy.

Aryl ist insbesondere carbocyclisches Aryl, z.B. Phenyl, ferner Naphthyl.

Arylniederalkyl ist insbesondere carbocyclisches Aryl, z.B. Aryl-$C_1$-$C_4$-alkyl, insbesondere Benzyl.

Carbocyclisches Aryl ist ferner Phenyl, welches durch ein bis drei Substituenten der Gruppe Niederalkyl, z.B. Methyl, Niederalkoxy, z.B. Methoxy, Trifluormethyl und Halogen substituiert sein kann.

Cycloalkyl ist vorzugsweise $C_5$-$C_7$-Cycloalkyl, z.B. Cyclopentyl, Cyclohexyl oder Cycloheptyl, vorzugsweise Cyclohexyl.

Pharmazeutisch annehmbare Salze sind vorzugsweise Säureadditionssalze von pharmazeutisch annehmbaren Säuren oder Basen, z.B. verdünnten starken Mineralsäuren, z.B. Chlorwasserstoff- oder Bromwasserstoffsäure, Schwefelsäure oder Phosphorsäure oder aliphatischen, aromatischen oder aromatisch-aliphatischen Carbon- oder Sulfonsäuren, z.B. Essigsäure, Propionsäure, Bernsteinsäure, Malonsäure, Glycolsäure, Milchsäure, Apfelsäure, Weinsäure, Gluconsäure, Citronensäure, Ascorbinsäure, Maleinsäure, Fumarsäure, Brenztraubensäure, Pamoasäure, Nicotinsäure, Methansulfonsäure, Ethansulfonsäure, Hydroxyethansulfonsäure, Benzolsulfonsäure, p-Toluolsulfonsäure oder Naphthalinsulfonsäure.

Die Verbindungen der Formel I haben wertvolle pharmakologische Eigenschaften, insbesondere sind sie antikonvulsiv wirksam. Die Verbindungen eignen sich daher zur Behandlung von Störungen und Krankheiten, die mit Konvulsionen verlaufen, z.B. Epilepsien.

Die pharmakologische Wirkung lässt sich beispielsweise in erprobten Testmodellen in-vivo an Säugern, z.B. Ratten, Mäusen oder Affen nachweisen. Die Verbindungen werden jeweils enteral oder parenteral, vorzugsweise oral oder transdermal, subcutan, intravenös oder intraperitoneal appliziert, z.B. mit Gelatinekapseln oder in der Form von wässrigen Suspensionen oder Lösungen. Der applizierte Dosisbereich liegt zwischen ca. 0,1 bis 100 mg/kg, vorzugsweise zwischen ca. 1,0 und 50 mg/kg, vorzugsweise zwischen ca. 5 und 30 mg/kg.

Die antikonvulsiven Eigenschaften der Verbindungen der Formel I lassen sich in geeichten Testanordnungen messen, welche zum Nachweis der antikonvulsiven Wirkung herangezogen werden können (Assays), z.B. im maximalen Elektroschocktest oder im Modell an der Ratte mit Pentylentetrazol (PTZ, Metrazol) Gaben initiierten Konvulsionen, z.B. gem. J. Pharmacol. Exper. Ther. 235, 95 (1985). Dabei wird die $ED_{50}$ bestimmt, welche die Dosis angibt, die 50 % der untersuchten Tiere gegen Konvulsionen schützt, welche durch Elektroschock oder durch Metrazol-Gaben verursacht werden.

Als repräsentatives Beispiel hat die Verbindung 5-Chlor-3-[1-(1H-imidazol-1-yl)ethyl]-1,2-benzisoxazol-hydrochlorid in der Ratte eine $ED_{50}$ von ca. 5 mg/kg p.o. gegen maximale Elektroschockkonvulsionen. Gegen Pentylentetrazol induzierte Konvulsionen in der Ratte hat diese Verbindung eine $ED_{50}$ von ca. 10

mg/kg p.o.

Aufgrund dieser günstigen pharmakologischen Eigenschaften, welche ein breites Spektrum von antikonvulsiven Eigenschaften belegen, eignen sich die Verbindungen der Formel I zur therapeutischen Verwendung bei der Behandlung von epileptischen Zuständen in Säugern, insbesondere bei Menschen.

Die Verbindungen der Formel I und deren Salze lassen sich anhand der folgenden Verfahren herstellen:

a) Die Verbindungen der Formel I, in der alle Symbole die angegebenen Bedeutungen haben, durch Kondensation einer Verbindung der Formel V

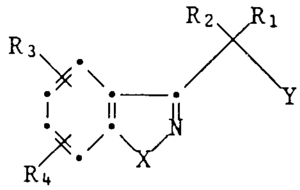

(V)

worin $R_1$-$R_4$ und X die genannten Bedeutungen haben, und Y eine reaktionsfähige, veresterte Hydroxygruppe darstellt, mit einer Verbindung der Formel VIa oder VIb

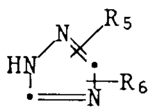

(VIa)

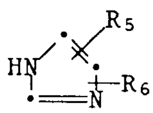

(VIb)

worin $R_5$ an einem Ring-C-Atom Wasserstoff, Niederalkyl oder Hydroxyniederalkyl, vorzugsweise in geschützter Form, und $R_6$ an einem Ring-C-Atom Wasserstoff oder Niederalkyl bedeutet, oder worin $R_5$ und $R_6$ zusammen $C_3$-$C_5$-Alkylen darstellen und dabei Z ein C-Atom darstellt und $R_5$ und $R_6$ zusammen dieses C-Atom und das nachbarständige C-Atom substituieren, und indem man gewünschtenfalls reaktive funktionelle Gruppen intermediär bei der Ausführung eines der genannten Verfahren schützt und anschliessend die freie Verbindung der Formel I isoliert und/oder eine erhältliche Verbindung der Formel I in eine andere Verbindung der Formel I umwandelt und/oder eine erhältliche freie Verbindung in ein Salz oder ein Salz in die freie Verbindung oder in ein anderes Salz umwandelt und/oder ein erhältliches Gemisch von Isomeren oder Racematen in die einzelnen Isomere oder Racematen auftrennt und/oder ein Racemat in die optischen Antipoden aufspaltet.

b) Eine Verbindung der Formel I, worin Z die Gruppe -CH= darstellt und die übrigen Symbole die im Anspruch 1 genannten Bedeutungen haben, indem man eine Verbindung der Formel V (wie unter Verf. a) definiert) mit einer Verbindung der Formel VII

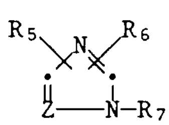

(VII)

worin $R_5$ Wasserstoff, Niederalkyl oder Hydroxyniederalkyl, $R_6$ Wasserstoff oder Niederalkyl oder $R_5$ und $R_6$ zusammen $C_3$-$C_5$-Alkylen darstellen und dabei Z ein C-Atom darstellt und $R_5$ und $R_6$ zusammen dieses C-Atom und das nachbarständige C-Atom substituieren, und $R_7$ eine übliche Aminoschutzgruppe darstellt, kondensiert und anschliessend die Aminoschutzgruppe abspaltet, vorzugsweise in-situ, und gewünschtenfalls reaktive funktionelle Gruppen intermediär bei der Ausführung eines der genannten Verfahren schützt und anschliessend die freie Verbindung der Formel I isoliert und/oder eine erhältliche Verbindung der Formel I in eine andere Verbindung der Formel I umwandelt und/oder eine erhältliche freie Verbindung in ein Salz oder ein Salz in die freie Verbindung oder in ein anderes Salz umwandelt und/oder ein erhältliches Gemisch von Isomeren oder Racematen in die einzelnen Isomere oder Racematen auftrennt und/oder ein Racemat in die optischen Antipoden aufspaltet.

c) Die Verbindungen der Formel I, worin Z die Gruppe =CH- darstellt und $R_1$ zusammen mit $R_5$ als

Substituent des C-Atoms in der Imidazolylgruppe $C_2$-$C_4$-Alkylen bedeuten, indem man eine Verbindung der Formel VIII

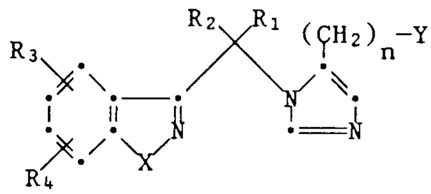

(VIII)

worin X, $R_3$ und $R_4$ wie weiter vorn definiert sind, $R_2$ die weiter vorn genannten Bedeutungen, ausser Niederalkylen gemeinsam mit $R_1$, hat und Y reaktives verestertes Hydroxy bedeutet, in Gegenwart einer Base zyklisiert und gewünschtenfalls reaktive funktionelle Gruppen intermediär bei der Ausführung eines der genannten Verfahren schützt und anschliessend die freie Verbindung der Formel I isoliert und/oder eine erhältliche Verbindung der Formel I in eine andere Verbindung der Formel I umwandelt und/oder eine erhältliche freie Verbindung in ein Salz oder ein Salz in die freie Verbindung oder in ein anderes Salz umwandelt und/oder ein erhältliches Gemisch von Isomeren oder Racematen in die einzelnen Isomere oder Racematen auftrennt und/oder ein Racemat in die optischen Antipoden aufspaltet.

Eine reaktionsfähige, veresterte Hydroxygruppe in den genannten Verfahrensvarianten ist beispielsweise eine Hydroxygruppe, welche durch eine starke Säure, insbesondere eine starke anorganische Säure wie Halogenwasserstoff, z.B. Chlor-, Brom- oder Jodwasserstoff, oder Schwefelsäure, oder durch eine starke organische Säure, beispielsweise eine Sulfonsäure, wie eine aliphatische oder aromatische Sulfonsäure, z.B. Methansulfonsäure, p-Toluolsulfonsäure oder 4-Brombenzolsulfonsäure, verestert ist. Eine solche reaktionsfähige, veresterte Hydroxygruppe ist insbesondere Halogen, z.B. Chlor, Brom oder Jod, oder durch aliphatische oder aromatische Gruppen substituiertes Sulfonyloxy, z.B. Mesyloxy oder Tosyloxy.

Die in den Ausgangsmaterialien, Zwischenstufen und in Verbindungen der Formel I vorhandenen funktionellen Gruppen, insbesondere die Carboxy-, Amino-, Hydroxy- und Sulfogruppen, sind gegebenenfalls durch solche Schutzgruppen (conventional protecting groups) geschützt, die üblicherweise in der Penicillin-, Cephalosporin- und Peptidchemie verwendet werden. Diese Schutzgruppen sollen die betreffenden funktionellen Gruppen gegen unerwünschte Nebenreaktionen wie Acylierungen, Verätherungen, Veresterungen, Oxydationen, Solvolyse etc. schützen und unter schonenden Reaktionsbedingungen leicht abspaltbar sein, das heisst unter Vermeidung von Spaltung z.B. solvolytischer, reduktiver, photolytischer oder auch enzymatischer Spaltung, sowie anderer Nebenreaktionen.

Der Schutz von funktionellen Gruppen mit solchen Schutzgruppen, die Schutzgruppen selbst sowie ihre Abspaltungsreaktionen, sind beispielsweise in "Protective Groups in Organic Chemistry", Plenum Press, London und New York 1973, in Greene, Th.W. "Protective Groups in Organic Synthesis", Wiley, New York 1981, in "The Peptides", Vol. I, Schröder and Lubke, Academic Press, London und New York 1965, sowie in "Methoden der organischen Chemie", Houben-Weyl, 4. Auflage, Bd. 15/I, Georg Thieme Verlag, Stuttgart 1974, beschrieben.

Die Kondensationsreaktion gemäss der Verfahrensvariante a) kann man analog den für N-Alkylierungen bekannten Methoden und Reaktionsbedingungen durchführen, z.B. in Gegenwart einer starken organischen Stickstoff-Base, z.B. Triäthylamin oder Pyridin in einem inerten Lösungsmittel, z.B. Acetonitril oder Methylenchlorid, bei Raumtemperaturen oder erhöhter Temperatur, z.B. einer Temperatur bis zum Siedepunkt des Reaktionsgemisches.

Die Kondensationsreaktion wird vorzugsweise in Gegenwart einer starken Base, z.B. einem Alkalimetallhydrid, z.B. Natrium- oder Kaliumhydrid, von einem Alkanolat, z.B. Natriummethanolat ode -ethanolat oder Kalium-tert-butanolat, oder einem Amid, z.B. Lithium-diisopropylamid, oder einer Alkyllithium-Verbindung, z.B. n-Butyllithium in inerten Lösungsmittel wie Hexan, Dimethylformamid oder Tetrahydrofuran, durchgeführt.

Verfahrensvariante a) eignet sich vorzugsweise zur Herstellung von Verbindungen der Formel I, worin $R_1$ und $R_2$ Wasserstoff bedeuten. Von Wasserstoff verschiedene Substituenten $R_1$ und $R_2$ lassen sich durch nachträgliche Umwandlung analog bekannter Substitutionsmethoden herstellen.

Ausgangsmaterialien der Formeln VIa und VIb mit Imidazol- und Triazol-Struktur sind bekannt oder können anhand bekannter Verfahren hergestellt werden.

Ausgangsmaterialien der Formel V sind bekannt oder lassen sich anhand bekannter Verfahren herstellen, z.B. gemäss Chimie Therapeutique 1972, 127-132, Phytochemistry 1971, 539, J. Heterocyclic Chem. 8, 397 (1971) und Chem. Pharm. Bull. 24, 632-643 (1976) für 1,2-Benzisoxazol-Derivate oder gemäss Chem.

Pharm. Bull. 26, 3888 (1978) für 1,2-Benzisothiazol-Derivate, indem man von 4-Hydroxycoumarinen oder 4-Hydroxythiocoumarinen ausgeht.

Die Kondensationsreaktion gemäss der Verfahrensvariante b) kann man analog den für N-Alkylierungen bekannten Methoden und Reaktionsbedingungen durchführen, z.B. analog den für die Herstellung von quaternären Ammoniumsalzen bekannten Reaktionsbedingungen, z.B. in einem inerten Lösungsmittel, z.B. Acetonitril oder Methylenchlorid, bei Raumtemperaturen oder erhöhter Temperatur, z.B. einer Temperatur bis zum Siedepunkt des Reaktionsgemisches.

Eine geeignete Aminoschutzgruppe für den freien Stickstoff der Imidazolylgruppe ist z.B. Trialkylsilyl wie Trimethylsilyl, Niederalkanoyl, z.B. Acetyl oder Formyl, Dimethylcarbamoyl, Triphenylmethyl oder tert-Butoxycarbonyl (BOC).

Das Alkylierungsverfahren erfolgt an dem ungeschützten N-Atom der Imidazolgruppe, wobei man eine quaternäre Verbindung bildet, welche man anschliessend in-situ von der Schutzgruppe befreit, z.B. mittels bekannter solvolytischer Abspaltungsmethoden in Gegenwart von Säure oder Base, insbesondere mit Ammoniak, wenn die Schutzgruppe Dimethylcarbamoyl ist. Anschliessend lässt sich die freie Verbindung der Formel I isolieren.

Das Zyklisierungsverfahren gemäss Verfahrensvariante c) erfolgt in Gegenwart einer starken Base, z.B. einem Alkalimetallhydrid, z.B. Natrium- oder Kaliumhydrid, von einem Alkanolat, z.B. Natriummethanolat oder Kalium-tert-butanolat, einem Alkalimetallamid, z.B. Lithiumdiisopropylamid, oder einer Alkyllithium-Verbindung, z.B. n-Butyllithium in einem inerten Lösungsmittel wie n-Hexan, Dimethylformamid oder Tetrahydrofuran.

Ausgangsmaterialien der Formel VIII lassen sich z.B. durch Halogenierungsverfahren der entsprechenden Hydroxy-Verbindung (Y = OH), z.B. durch Umsetzung mit einem Halogenierungsmittel wie Thionylchlorid herstellen, wobei man Verbindungen der Formel VIII erhält, worin Y Chlor darstellt. Die Hydroxyverbindung, welche einer Verbindung der Formel I entspricht, worin $R_5$ Hydroxyalkyl darstellt, erhält man nach Ausführung des Verfahrens b). Die für dieses Verfahren benötigte Hydroxyalkylimidazol-Verbindung, worin die Hydroxygruppe, z.B. durch eine Trimethylsilylgruppe geschützt ist, erhält man durch Schutz des 1-Stickstoffatoms der Imidazolgruppe durch Acylierung mit Dimethylcarbamoylchlorid und anschliessender Alkylierung mit dem Zwischenprodukt der Formel V.

Nach Ausführung einer der genannten Verfahrensvarianten lässt sich eine erhältliche Verbindung der Formel I in eine andere Verbindung der Formel I umwandeln.

Beispielsweise lässt sich eine Verbindung der Formel I, worin $R_1$ und $R_2$ Wasserstoff darstellen, in eine Verbindung der Formel I umwandeln, worin eine der Gruppen $R_1$ und $R_2$ Niederalkyl bedeutet, und zwar durch Alkylierung einer Verbindung mit einem reaktiven Esterderivat eines Niederalkanols, z.B. einem Niederalkylhalogenid wie Methyljodid, in Gegenwart einer starken Base, z.B. einem Alkalimetallamid, z.B. Lithiumdiisopropylamid, oder den anderen weiter vorn für Alkylierungsverfahren genannten starken Basen.

Ebenfalls kann man eine Verbindung der Formel I, worin eine der Gruppen $R_1$ und $R_2$ Niederalkyl bedeutet, in eine Verbindung der Formel I umwandeln, worin beide Gruppen $R_1$ und $R_2$ Niederalkyl bedeuten. Dabei kann die Alkylierung mit einem zusätzlichen Aequivalent des Alkylierungsmittels erfolgen. Diese Umwandlungsverfahren mittels Alkylierung eignen sich auch zur Herstellung von Verbindungen der Formel I, worin eine der Gruppen oder beide Gruppen $R_1$ und $R_2$ Arylniederalkyl, z.B. Benzyl, Niederalkenyl, z.B. Allyl, oder Niederalkinyl, z.B. Propargyl, bedeuten.

Eine Verbindung der Formel I, worin $R_1$ und $R_2$ Wasserstoff bedeuten, lässt sich auch mittels reduktiver Alkylierung in eine Verbindung der Formel I umwandeln, worin beide oder eine Gruppe $R_1$ und $R_2$ Niederalkyl bedeutet.

Dies kann z.B. durch Umsetzung mit einer Aldehydverbindung wie Acetaldehyd in Gegenwart einer der genannten starken Basen, anschliessender Dehydrierung des gebildeten Alkohols und Reduzierung des Niederalkylidinyl-Derivatsdurch Hydrierung erfolgen unter Anwendung der üblichen für solche reduktiven Alkylierungen bekannten Reaktionsbedingungen.

Eine Verbindung der Formel I, worin $R_1$ und $R_2$ Wasserstoff bedeuten, lässt sich in eine Verbindung der Formel I umwandeln, worin $R_1$ und $R_2$ Niederalkylen darstellen, und zwar durch Umsetzung eines reaktionsfähigen Esterderivats eines terminal Niederalkylendiols, z.B. Aethylenoxid, indem man jeweils in einem Schritt zwei Aequivalente oder in zwei Schritten jeweils ein Aequivalent der genannten starken Basen wie Natrium- oder Kaliumhydrid in einem der genannten inerten Lösungsmittel zusetzt.

Die oben genannten nachträglichen Reaktionen werden nach an sich bekannten Methoden, gegebenenfalls in Gegenwart von Lösungsmitteln, vorzugsweise solchen, welche gegenüber den Reagenzien inert sind und diese lösen, Katalysatoren, Kondensations- oder anderen oben genannten Reagenzien, gegebenenfalls in einer inerten Atmosphäre, unter Kühlung, bei Zimmertemperatur oder bei erhöhter Temperatur, vorzugsweise beim Siedepunkt des verwendeten Lösungsmittels, bei normalem oder erhöhtem Druck, durchgeführt.

Die Erfindung betrifft ebenfalls sämtliche Abänderungen des vorliegenden Verfahrens, wonach ein auf irgendeiner Stufe des Verfahrens erhaltenes Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin ein Ausgangsstoff in Form eines Salzes oder optisch reinen Antipoden verwendet wird. Immer wenn gewünscht, werden die oben genannten Verfahren erst durchgeführt, nachdem alle potentiell störenden, reaktionsfähigen funktionellen Gruppen in geeigneter Weise geschützt sind, z.B. wie oben und in den Beispielen illustriert.

In den genannten Reaktionen werden vorteilhafterweise solche Ausgangsstoffe verwendet, welche zu den weiter vorn als besonders bevorzugt beschriebenen Verbindungen führen.

Die Erfindung betrifft auch neue Ausgangsstoffe und Verfahren zu ihrer Herstellung.

Abhängig von den gewählten Ausgangsstoffen und Methoden können die neuen Verbindungen in Form von Isomeren oder als Isomerengemische vorliegen, zum Beispiel als geometrische Isomere oder Mischungen davon (cis oder trans), als optische reine Antipoden, Racemate oder Diastereomere.

Erhaltene geometrische oder diastereomere Isomerengemische der obigen Verbindungen oder Zwischenprodukte können nach an sich bekannten Methoden in die einzelnen racemischen oder optisch aktiven Isomeren getrennt werden, z.B. durch fraktionierte Destillation, Kristallisation und/oder Chromatographie. Racemische Produkte können gleichfalls in die optischen Antipoden gespalten werden, z.B. durch Trennung ihrer diastereomeren Salze, wie gemäss J. Org. Chem. 43, 3803 (1978), z.B. durch fraktionierte Kristallisation von d- und ℓ-(Tartraten, Mandelaten, Camphersulfaten oder 1-Naphthyl-1-äthylisocyanaten) von Verbindungen, die eine basische, salzbildende Gruppe haben, oder von d- und ℓ-(α-Methylbenzylamin, Cinchonidin,, Chinin, Chinidin, Ephedrin, Dehydroabietylamin, Brucin oder Strychnin)-Salzen von Verbindungen, die eine saure,salzbildende Gruppe besitzen.

Bevorzugt wird jeweils das wirksamere Isomere und/oder der wirksamere Antipode der erfindungsgemässen Verbindungen isoliert.

Die Verbindungen der vorliegenden Erfindung werden entweder in freier oder in Form ihrer Salze erhalten. Jede erhaltene Base kann in das entsprechende Säureadditionssalz umgewandelt werden, vorzugsweise unter Verwendung einer therapeutisch annehmbaren Säure oder eines Anionenaustauschers. Erhaltene Salze können in die entsprechenden freien Basen umgewandelt werden, zum Beispiel unter Verwendung einer stärkeren Base, beispielsweise eines Metall- oder Ammoniumhydroxids oder eines basischen Salzes, z.B. eines Alkalimetallhydroxids oder -carbonats, oder eines Kationenaustauschers. Diese oder andere Salze, etwa Pikrate, können auch zur Reinigung der erhaltenen Basen verwendet werden, indem man die Basen in Salze überführt, diese abtrennt und reinigt und aus den Salzen wiederum die Base freisetzt. Infolge der engen Beziehung zwischen den freien Verbindungen und ihren Salzen sind in diesem Zusammenhang unter freien Verbindungen sinn- und zweckmässig gegebenenfalls immer auch die entsprechenden Salze zu verstehen.

Die Verbindungen und ihre Salze können auch in Form ihrer Hydrate erhalten werden, oder sie können andere zur Kristallisation verwendete Lösungsmittel einschliessen.

Die erfindungsgemässen pharmazeutischen Präparate eignen sich zur enteralen, wie oralen oder rektalen, transdermalen oder parenteralen Verabreichung an Säugetieren, inkl. Menschen, zur Behandlung von Zuständen, Störungen und Krankheiten, welche mit Konvulsionen als äusserem Erscheinungsbild verlaufen z.B. Epilepsien wie Grand Mal oder Petit Mal sowie generell epileptischen Zuständen. Diese Präparate enthalten eine wirksame Menge einer Verbindung der Formel I oder eines pharmazeutisch annehmbaren Salzes davon und zwar ohne Beimengung oder in Kombination mit einem oder mehreren pharmazeutisch annehmbaren Trägermaterialien.

Die pharmakologisch aktiven Verbindungen der Erfindung sind bei der Herstellung von pharmazeutischen Zusammensetzungen verwendbar, die eine wirksame Menge derselben in Verbindung oder Beimischung mit Excipienten oder Trägern, die für die enterale oder parenterale Anwendung geeignet sind, umfassen. Bevorzugt sind Tabletten und Gelatinekapseln, die den aktiven Bestandteil zusammen mit a) Verdünnungsmitteln, z.B. Lactose, Dextrose, Sucrose, Mannit, Sorbit, Cellulose und/oder Glycin, b) Gleitmitteln, z.B. Siliciumdioxid, Talk, Stearinsäure, deren Magnesium- oder Calciumsalz und/oder Polyethylenglycol, für Tabletten auch c) Bindemitteln, z.B. Magnesiumaluminiumsilicat, Stärkepaste, Gelatine, Tragant, Methylcellulose, Natriumcarboxymethylcellulose und/oder Polyvinylpyrrolidon, gewünschtenfalls d) Zerteilungs- bzw. Desintegrationsmitteln, z.B. Stärken, Agar, Alginsäure oder deren Natriumsalz, oder schäumenden Mischungen und/oder e) Absorbentien, farbgebenden Mitteln, Geschmacksstoffen und süssenden Mitteln umfassen. Injizierbare Präparate sind vorzugsweise wässrige isotonische Lösungen oder Suspensionen, und Suppositorien werden vorteilhaft aus Fettemulsionen oder -suspensionen hergestellt.

Diese Zusammensetzungen können sterilisiert werden und/oder Adjuvanzien, wie Konservierungs-, Stabilisierungs-, Netz- oder Emulgiermittel, Lösungspromotoren, Salze für die Regulierung des osmotischen Drucks und/oder Puffer, enthalten. Zusätzlich können sie auch andere therapeutisch wertvolle Substanzen enthalten. Diese Präparate werden nach herkömmlichen Misch-, Granulier- bzw. Ueberzugsmethoden hergestellt und enthalten etwa 0,1 bis 75 %, vorzugsweise etwa 1 bis 50 %, des aktiven Bestandteils.

Geeignete Formulierungen für die transdermale Anwendung enthalten eine wirksame Menge einer Verbindung der Formel I zusammen mit Trägermaterial. Geeignete Trägermaterialien enthalten pharmazeutisch annehmbare Hilfsstoffe, welche den Durchgang durch die Haut ermöglichen. Insbesondere haben transdermale therapeutische Systeme die Form eines Pflasters mit einer Schutzschicht, einem Wirkstoffreservoir, gegebenenfalls mit Trägermaterialien, und einer die Abgabe bestimmenden Kontrollschicht, durch welche der Wirkstoff auf die Haupt mit kontrollierter und bestimmbarer Geschwindigkeit über einen längeren Zeitabschnitt abgegeben wird. Das System hat gegebenenfalls noch eine Klebschicht.

Die vorliegende Erfindung betrifft ebenfalls ein therapeutisches Verfahren zur Behandlung von konvulsiven Störungen wie Epilepsie in Säugern inkl. Menschen, indem man beispielsweise eine therapeutisch wirksame Menge einer Verbindung der Formel I oder eines pharmazeutisch annehmbaren Salzes davon, vorzugsweise in Form einer der weiter von beschriebenen pharmazeutischen Zusammensetzungen, angewendet wird.

Die applizierbare Wirkstoffdosis ist speziesabhängig sowie vom Körpergewicht, dem Alter und individuellen Zustand und der Darreichungsform abhängig.

Die Einheitsdosis für einen Säuger, z.B. Mensch, von ca. 50 bis 70 kg Gewicht beträgt zwischen 15 und 200 mg des Wirkstoffs.

Die folgenden Beispiele sollen die Erfindung erläutern und keine Einschränkungen darstellen. Die Temperaturen sind in Celsiusgraden angegeben, und sämtliche Teile sind in Form von Gewichtsteilen angegeben. Wenn nicht anders erwähnt, werden sämtliche Verdampfungen unter vermindertem Druck vorzugsweise zwischen etwa 15 und 100 mg Hg durchgeführt.

Beispiel 1:

a) 5-Chlor-3-(1H-imidazol-1-ylmethyl)-1,2-benzisoxazol

276,6 g (6,92 Mol) Natriumhydrid-Suspension in Mineralöl (60%ig) wird in mehreren Portionen eine Stunde lang zu einer Lösung von 471 g (6,92 Mol) Imidazol in 4 l Dimethylformamid gegeben. Man lässt die Suspension bei Raumtemperatur eine Stunde lang rühren. Anschliessend setzt man eine Lösung von 1,57 kg (6,37 Mol) 3-Brommethyl-5-chlor-1,2-benzisoxazol in 3 l Dimethylformamid 30 Minuten hinzu, wobei die Temperatur von 24° auf 68° ansteigt. Danach lässt man das Gemisch zwei Stunden lang bei 90° rühren. Man engt das Gemisch ein, nimmt den Rückstand in 4 l Methylenchlorid auf und wäscht die gebildete Suspension zweimal mit 2 l Wasser, wobei sich der feste Rückstand löst. Man trocknet die organische Phase über Natriumsulfat, filtriert, engt ein und erhält die Titelverbindung mit einem Schmelzpunkt von 100-102° für die freie Verbindung und 201-203° für das Hydrochlorid.

Herstellung des Ausgangsmaterials:

Man erhitzt 3,39 kg (11,7 Mol) 2-Brom-2-(5-chlor-1,2-bromisoxazol-3-yl)essigsäure, suspendiert in 12 l Toluol, 18 Stunden lang auf 96°. Man lässt das Gemisch auf Raumtemperatur abkühlen, wäscht dieses mit Wasser, gesättigter wässriger Natriumhydrogencarbonat-Lösung, trocknet über Natriumsulfat und filtriert. Aus dem Filtrat erhält man durch Eindampfen das rohe 3-Brommethyl-5-chlor-1,2-benzisoxazol mit einem Schmelzpunkt von 83-85°.

Die alpha-Brom-5-chlor-1,2-benzisoxazol-3-essigsäure erhält man gemäss der Vorschrift in Chimie Therapeutique 1972, 127 aus der entsprechenden 5-Chlor-1,2-benzisoxazol-3-essigsäure.

b) 3-(1H-Imidazol-1-ylmethyl)-1,2-benzisoxazol

3,4 g (0,071 Mol) Natriumhydrid-Suspension in Mineralöl (50%ig) wird durch Auswaschen mit n-Hexan vom Mineralöl befreit und in 100 ml Dimethylformamid resuspendiert. Man lässt die Suspension gut rühren und hält diese angenähert konstant bei Raumtemperatur, während man tropfenweise 4,8 g (0,071 Mol) Imidazol in 25 ml Dimethylformamid hinzufügt. Man lässt das Reaktionsgemisch eine Stunde lang bei Raumtemperatur rühren und fügt 15,0 g (0,071 Mol) 3-Brommethyl-1,2-benzisoxazol auf einmal hinzu. Man erhitzt das Gemisch acht Stunden lang auf ca. 85°, dampft das Lösungsmittel ab, nimmt den Rückstand in Methylenchlorid auf und wäscht diesen mit Wasser. Man trocknet die organische Lösung über Natriumsulfat, filtriert, dampft ein und kristallisiert die gebildete Titelverbindung aus Isopropanol um. Schmelzpunkt 52-55° für die freie Verbindung und 145-146° für das Hydrochlorid, welches man durch Behandeln einer Lösung der freien Verbindung in Isopropanol mit einer chlorwasserstoffhaltigen Aetherlösung erhält.

Das Ausgangsmaterial 3-Brommethyl-1,2-benzisoxazol kann man nach der Vorschrift in Chem. Pharm. Bull 24, 632 (1972) herstellen.

c) Analog kann man durch Kondensation von 3-Brommethyl-1,2-benzisoxazol mit 1,2,4-Triazol das entsprechende 3-(1H-1,2,4-Triazol-1-ylmethyl)-1,2-benzisoxazol herstellen. Schmelzpunkt: 102-104°.

Die folgenden Verbindungen lassen sich analog unter Verwendung der substituierten Ausgansmaterialien herstellen:

d) 3-(2-Methyl-1H-imidazol-1-ylmethyl)-1,2-benzisoxazol, Schmelzpunkt: 84-86°;
e) 3-(4-Methyl-1H-imidazol-1-ylmethyl)-1,2-benzisoxazol-hydrochlorid, Schmelzpunkt: 225-227°;
f) 5-Brom-3-(1H-imidazol-1-ylmethyl)-1,2-benzisoxazol-hydrochlorid,Schmelzpunkt: 230-232°;
g) 5-Brom-3-(1H-1,2,4-triazol-1-ylmethyl)-1,2-benzisoxazol-hydrochlorid, Schmelzpunkt: 187-189°;
h) 5-Brom-3-(4-methyl-1H-imidazol-1-ylmethyl)-1,2-benzisoxazol-hydrochlorid, Schmelzpunkt: 188-190°;
i) 5-Chlor-3-(1H-1,2,4-triazol-1-ylmethyl)-1,2-benzisoxazol-hydrochlorid, Schmelzpunkt: 173-175°;
j) 5-Methyl-3-(1H-imidazol-1-ylmethyl)-1,2-benzisoxazol-hydrochlorid, Schmelzpunkt: 178-180°; Ausgangsmaterial: 3-Brommethyl-5-methyl-1,2-benzisoxazol, NMR ($CH_2$-methylen): 5,35 (s) ppm.
k) 5-Methyl-3-(1H-1,2,4-triazol-1-ylmethyl)-1,2-benzisoxazol-hydrochlorid, Schmelzpunkt: 172-174°;
l) 5-Fluor-3-(1H-imidazol-1-ylmethyl)-1,2-benzisoxazol-hydrochlorid, Schmelzpunkt: 179-181°; Ausgangsmaterial: 3-Brommethyl-5-fluor-1,2-benzisoxazol, Schmelzpunkt: 48-50°;
m) 5-Fluor-3-(1H-1,2,4-triazol-1-ylmethyl)-1,2-benzisoxazol-hydrochlorid, Schmelzpunkt: 160-162°;
n) 6-Chlor-3-(1H-imidazol-1-ylmethyl)-1,2-benzisoxazol-hydrochlorid, Schmelzpunkt: 224-226°; Ausgangsmaterial: 3-Brommethyl-6-chlor-1,2-benzisoxazol, Schmelzpunkt: 80-82°;
o) 6-Chlor-3-(1H-1,2,4-triazol-1-ylmethyl)-1,2-benzisoxazol-hydrochlorid, Schmelzpunkt: 175-177°;
p) 6-Chlor-3-(4-methyl-1H-imidazol-1-ylmethyl)-1,2-benzisoxazol-hydrochlorid, Schmelzpunkt: 185-187°;
q) 5-Phenyl-3-(1H-imidazol-1-ylmethyl)-1,2-benzisoxazol-hydrochlorid, Schmelzpunkt: 198-200°, Ausgangsmaterial: 3-Brommethyl-5-phenyl-1,2-benzisoxazol, Schmelzpunkt: 87-90°;
r) 5,6-Dimethyl-3-(1H-imidazol-1-ylmethyl)-1,2-benzisoxazol-hydrochlorid, Schmelzpunkt: 223-224°; Ausgangsmaterial: 3-Brommethyl-5,6-dimethyl-1,2-benzisoxazol, Schmelzpunkt: 41-44°C, hergestellt aus 6,7-Dimethyl-4-hydroxycoumarin, J. Med. Chem. 18, 391 (1975);
s) 5-Chlor-3-(4-methyl-1H-imidazol-1-ylmethyl)-1,2-benzisoxazol-hydrochlorid, Schmelzpunkt: 228-230°;
t) 5-Fluor-3-(4-methyl-1H-imidazol-1-ylmethyl)-1,2-benzisoxazol-hydrochlorid, Schmelzpunkt: 240-242°;
u) 3-(4,5-Dimethyl-1H-imidazol-1-ylmethyl)-1,2-benzisoxazol-hydrochlorid, Schmelzpunkt: 197-198°;
v) 5-Phenyl-3-(1H-1,2,4-triazol-1-ylmethyl)-1,2-benzisoxazol-hydrochlorid, Schmelzpunkt: 142-144°.

Beispiel 2:

Folgende Verbindungen lassen sich analog der im Beispiel 1 beschriebenen Methodik herstellen:

a) 3-(1H-Imidazol-1-ylmethyl)naphth-[2,1-d]isoxazol-hydrochlorid, Schmelzpunkt: 230-232°; Ausgangsmaterial: 3-Brommethylnapth-[2,1-d]isoxazol, Schmelzpunkt: 100-103°, hergestellt aus den entsprechenden benzokondensierten 4-Hydroxycoumarin, J. Het. Chem. 18, 587 (1981);
b) 3-(1H-Imidazol-1-ylmethyl)naphth-[2,3-d]isoxazol-hydrochlorid, Schmelzpunkt: 244-246°; Ausgangsmaterial: 3-Brommethylnaphth-[2,3-d]isoxazol, Schmelzpunkt: 111-113°, hergestellt aus dem entsprechenden benzokondensierten 4-Hydroxycoumarin, Indian J. Chem. 11, 115 (1973);
c) 6,7-Dihydro-3-(1H-imidazol-1-ylmethyl)-5H-indeno[5,6-d]isoxazol-hydrochlorid, Schmelzpunkt: 207-208°; Ausgangsmaterial: 6,7-dihydro-3-brommethyl-5H-indeno-[5,6-d]isoxazol, Schmelzpunkt: 69-71°, hergestellt aus dem entsprechenden cyclopenta-anellierten 4-Hydroxycoumarin.

Das cyclopenta-anellierte 4-Hydroxycoumarin wird folgendermassen hergestellt:
5-Acetoxyindan (Chemical Abstracts, 77, 126112w) wird zu 2-Acetyl-5-indanol durch Fries Reaktion umgelagert (siehe R. Martin et al., Monatsh. 81, 111 (1980)). Letzteres wird in das entsprechende 4-Hydroxycoumarin-Derivat gemäss Link et al., J. Med. Chem., 14, 167 (1971), überführt.

Beispiel 3:

a) 3-(1-(1H-Imidazol-1-ylmethyl)-ethyl)-1,2-benzisoxazol

Zur Herstellung von Lithiumdiisopropylamid wird 9,2 ml einer 2,1 molaren Lösung von n-Butyllithium in Hexan (0,019 Mol) zu einer Lösung von 2,1 g (0,021 Mol) Diisopropylamin in 40 ml wasserfreiem Tetrahydrofuran bei -40° zugetropft. Man lässt das Gemisch zehn Minuten lang bei dieser Temperatur stehen und giesst das noch kalte Gemisch zu einer gerührten auf -60° gekühlten Lösung von 3,7 g (0,019 Mol) 3-(1H-Imidazol-1-ylmethyl)-1,2-benzisoxazol in 40 ml wasserfreiem Tetrahydrofuran. Das tief rot gefärbte Reaktionsgemisch wird bei -60° dreissig Minuten lang gerührt und dazu werden 4,1 g

(0,029 Mol) Iodomethan in einer Portion hinzugefügt. Das Gemisch wird bei -60° eine Stunde lang und anschliessend zwei Stunden lang ohne äussere Kühlung gerührt. Man setzt je 100 ml Wasser und Essigsäureethylester hinzu, trennt die organische Phase ab und extrahiert diese zweimal mit je 30 ml 3 N Salsäurelösung. Man macht die vereinigten Säureextrakte mit 2 N Natriumhydroxidlösung basisch auf pH 8 und extrahiert das Produkt wiederum mit 2 x 50 ml Essigsäureethylester. Man trocknet die kombinierten Extrakte über Natriumsulfat, filtriert und dampft ein, worauf man ein Oel erhält, das sich beim Stehenlassen verfestigt. Man erhält die rohe Titelverbindung nach Umkristallisieren aus Isopropanol mit einem Schmelzpunkt von 62-64°. Durch Behandeln einer Lösung der freien Verbindung in warmen Isopropanol mit einer chlorwasserstoffhaltigen Aetherlösung erhält man das Hydrochlorid mit einem Schmelzpunkt von 163-165°.

Analog werden folgende Verbindungen hergestellt:

b) 5-Chlor-3-[1-(1H-imidazol-1-yl)ethyl]-1,2-benzisoxazol-hydrochlorid, Schmelzpunkt: 168-170°;
c) 3-[1-(5-Methyl-1H-imidazol-1-yl)ethyl]-1,2-benzisoxazol-hydrochlorid, Schmelzpunkt: 215-217°;
d) 3-[1-(1H-1,2,4-Triazol-1-yl)ethyl]-1,2-benzisoxazol-hydrochlorid, Schmelzpunkt: 153-155°;
e) 3-[1-(1H-Imidazol-1-yl)-1-propyl]-1,2-benzisoxazol-hemisuccinat, Schmelzpunkt: 108-110°;
f) 3-[1-(1H-Imidazol-1-yl)-1-pentyl]-1,2-benzisoxazol-oxalat, Schmelzpunkt: 68-70°;
g) 3-[1-(1H-Imidazol-1-yl)-2-methyl-1-propyl]-1,2-benzisoxazol-citrat, Schmelzpunkt: 65-68°;
h) 3-[1-(1H-Imidazol-1-yl)-1-butyl]-1,2-benzisoxazole, Schmelzpunkt: 69-70°;
i) 3-[1-(1H-Imidazol-1-yl)-1-but-3-enyl]-1,2-benzisoxazol, Schmelzpunkt: 79-80°;
j) 3-[1-(1H-1,2,4-Triazol-1-yl)-1-propyl]-1,2-benzisoxazol, Schmelzpunkt: 68-70°;
k) 5-Chlor-3-[1-(1H-imidazol-1-yl)-1-propyl]-1,2-benzisoxazol-maleat, Schmelzpunkt: 123-125°;
l) 5-Chlor-3-[1-(1H-imidazol-1-yl)-2-methyl-1-propyl]-1,2-benzisoxazolmaleat, Schmelzpunkt: 88-90°;
m) 3-[2-(1H-Imidazol-1-yl)-2-propyl]-1,2-benzisoxazol-hydrochlorid, Schmelzpunkt: 211-213°, durch Verwendung von 3-[1-(1H-Imidazol-1-yl)ethyl]-1,2-benzisoxazol als Ausgangsmaterial;
n) 3-[1-(1H-Imidazol-1-yl)-2-phenylethyl]-1,2-benzisoxazol, Schmelzpunkt: 142-143°.

Beispiel 4:

a) 5-Chlor-3-[1-(1H-imidazol-1-yl)-ethyl]-benzisoxazol

Eine Lösung von 500 g (2,14 Mol) 5-Chlor-3-(1H-imidazol-1-ylmethyl)-1,2-benzisoxazol in 3,5 l Dimethylformamid wird auf -35° gekühlt und zu einer ebenfalls auf -35° gekühlten Lösung von 266 g (2,37 Mol) Kalium-tert-butanolat in in 3,5 l Dimethylformamid gegeben. Dabei färbt sich das Reaktionsgemisch tiefrot. Man rührt das Gemisch fünf Minuten lang bei -35° und kühlt zehn Minuten lang auf -55°. Bei dieser Temperatur gibt man 433 g (3,05 Mol) Methyljodid in einer Portion hinzu. Die Reaktionstemperatur steigt bis auf -22°, fällt wieder ab, worauf bei -26° sich das Reaktionsgemisch entfärbt, festes Kaliumbromid sich abscheidet und die Temperatur wieder auf -22° ansteigt. Das Gemisch kühlt wieder schnell auf -55° Badtemperatur ab, worauf man noch dreissig Minuten lang bei dieser Temperatur rühren lässt. Anschliessend lässt man innerhalb von 1,5 Stunden die Temperatur langsam auf 0° ansteigen. Man verdünnt das Reaktionsgemisch mit 35 l Wasser und extrahiert die wässrige Phase mit 20 l Essigsäureethylester. Nach Abtrennen der organischen Phase wäscht man diese sechsmal mit je 6 l Wasser, trocknet über 5 kg Natriumsulfat, filtriert und engt ein. Man digeriert den öligen Rückstand in Petroläther bei Raumtemperatur und entfernt den gebildeten Rückstand. Nach dem Eindampfen digeriert man mehrmals in je drei Litern Ether, trocknet die vereinigten Aetherextrakte über 500 g Natriumsulfat und filtriert. Das Filtrat wird mit 500 ml einer 6 N chlorwasserstoffhaltigen Etherlösung angesäuert. Die gebildete Suspension wird anschliessend 30 Minuten gerührt. Die gebildete Suspension wird anschliessend 30 Minuten gerührt und filtriert. Man wäscht den Filterrückstand zweimal mit je 500 ml Ether und digeriert unter Rückflussbedingungen eine Stunde lang in 2 l Aceton. Man lässt die Suspension auf Raumtemperatur abkühlen und filtriert ab. Man wäscht den Filterrückstand zweimal mit je 500 ml Aceton und zweimal mit je 500 ml Ether und trocknet das Produkt achtzehn Stunden bei 60° und vermindertem Druck, worauf man das Hydrochlorid der Titelverbindung mit einem Schmelzpunkt von 161-166° erhält. Durch wiederholtes Reinigen und Umkristallisieren erhält man dieses Hydrochlorid mit einem Schmelzpunkt von 168-169°.

Beispiel 5: 5-Chlor-3-(5-methyl-1H-imidazol-1-ylmethyl)-1,2-benzisoxazol

2,5 g (0,01 Mol) 3-Brommethyl-5-chlor-1,2-benzisoxazol und 1,84 g (0,012 Mol) 1-Dimethylcarbamoyl-4-methylimidazol werden unter Rückflussbedingungen in 25 ml Dimethylformamid 72 Stunden lang gerührt. Man kühlt das Gemisch auf 5° ab und leitet fünf Minuten Ammoniakgas ein. Danach lässt man das

Gemisch eine Stunde lang bei Raumtemperatur stehen. Man dampft ein und nimmt den Rückstand in 25 ml Methylenchlorid auf. Man wäscht mit Wasser, trocknet die organische Phase über Natriumsulfat, filtriert und dampft ein, worauf man die Titelverbindung als öligen Rückstand erhält. Durch Behandeln einer Lösung des Rückstands in Aceton mit einer chlorwasserstoffhaltigen Aetherlösung erhält man das entsprechende Hydrochlorid mit einem Schmelzpunkt von 245-247°.

Herstellung des Ausgangsmaterials:

Eine Lösung von 17,0 g (0,025 Mol) 4-Methylimidazol, 27,0 g (0,25 Mol) N,N-Dimethylcarbamoylchlorid und 30 g (0,30 Mol) Triethylamin in 125 ml Acetonitril wird 20 Stunden unter Rückflussbedingungen erhitzt. Man lässt das Reaktionsgemisch auf Raumtemperatur abkühlen, verdünnt mit einem Liter Ether und filtriert. Man dampft das Filtrat ein und destilliert den Rückstand unter vermindertem Druck, worauf man bei 0,35 mm Hg 1-Dimethylcarbamoyl-4-methylimidazol mit einem Siedepunkt von 104-106° erhält.

b) 3-[(5-(3-Hydroxypropyl)-1H-imidazol-1-yl)methyl]-1,2-benzisoxazol

6,7 g (0,025 Mol) 1-Dimethylcarbamoyl-4-(3-trimethylsilyloxypropyl)imidazolund 5,3 g (0,025 Mol) 3-Brommethyl-1,2-benzisoxazol in 30 ml wasserfreiem Acetonitril werden 72 Stunden lang unter Rückflussbedingungen erhitzt. Man kühlt das Reaktionsgemisch auf Raumtemperatur ab und setzt 30 ml einer methanolischen Lösung enthaltend 3 g (0,055 Mol) Natriummethanolat hinzu. Man erhitzt das Reaktionsgemisch 26 Stunden lang unter Rückflussbedingungen, dampft ein und nimmt den Rückstand in 30 ml einer 1 N Salzsäurelösung auf. Man lässt drei Stunden lang bei Raumtemperatur stehen und macht anschliessend das Gemisch durch Zusatz von 50%iger wässriger Kaliumhydroxidlösung basisch (pH = 9). Man extrahiert das Reaktionsgemisch mit Methylenchlorid, wäscht mit Wasser, trocknet die organische Phase über Natriumsulfat, filtriert und dampft ein, worauf man die rohe Titelverbindung als öligen Rückstand erhält, welche man durch Verreiben mit Aceton reinigt. Schmelzpunkt der freien Verbindung: 129-131°. Die freie Verbindung bildet ein Hydrochloridhalbhydrat mit einem Schmelzpunkt von 155-157°, welches man durch Behandeln einer Lösung in Aceton mit einer chlorwasserstoffhaltigen Aetherlösung erhält.

Herstellung des Ausgangsmaterials:

Durch Kondensation von 4-(3-Hydroxypropyl)-imidazol mit N,N-Dimethylcarbamoylchlorid stellt man analog wie weiter vorn beschrieben 1-Dimethylcarbamoyl-4-(3-hydroxypropyl)-imidazol (NMR-Imidazol-Protonen: 2,15 (s) und 2,95 (s) ppm) her.

19,8 g (0,10 Mol) 1-Dimethylcarbamoyl-4-(3-hydroxypropyl)-imidazol und 15 g (0,15 Mol) Triethylamin werden in 100 ml Acetonitril gelöst, gerührt und auf 0° abgekühlt. Anschliessend wird tropfenweise 11,9 g (0,11 Mol) Trimethylchlorsilan hinzugesetzt. Man rührt das Gemisch eine Stunde lang bei 0° und lässt anchliessend 15 Stunden lang auf Raumtemperatur erwärmen. Man verdünnt mit 500 ml Ether, filtriert und dampft ein, worauf man die silylierte Verbindung als öligen Rückstand erhält. NMR-IMidazol-Protonen: 2,2 (s) und 3,05 (s) ppm.

Beispiel 6: 3-(5,6,7,8-Tetrahydroimidazo-[1,5-a]pyridin-5-yl)-1,2-benzisoxazol

Eine Suspension von 1,9 g (0,006 Mol) von 3-[5-(3-Chlorpropyl)-1H-imidazol-1-ylmethyl]-1,2-benzisoxazol in 25 ml wasserfreiem Tetrahydrofuran wird unter Stickstoffatmosphäre auf 0° gekühlt. Man setzt in mehreren Portionen 1,7 g (0,015 Mol) Kalium-tert-butanolat hinzu und rührt drei Stunden lang bei 0-5°. Anschliessend säuert man durch Zugabe von 0,5 ml Eisessig an, dämpft ein und nimmt den Rückstand in 20 ml Methylenchlorid auf. Diesen wäscht man mit 10 ml gesättigter wässriger Natriumhydrogencarbonat-Lösung und mit 10 ml Wasser. Man trocknet die Lösung über Natriumsulfat, filtriert und dampft ein, worauf man die rohe Titelverbindung erhält, welche man durch Behandeln einer isopropanolischen Lösung mit einer chlorwasserstoffhaltigen Aetherlösung in das Hydrochlorid mit einem Schmelzpunkt von 250-252° überführt.

Herstellung des Ausgangsmaterials:

Man lässt eine Lösung von 2,4 g (0,02 Mol) Thionylchlorid in 15 ml Methylenchlorid zu einer Suspension von 2,6 g (0,01 Mol) 3-[5-(3-Hydroxypropyl)-1H-imidazol-1-ylmethyl]-1,2-benzisoxazol in 15 ml Methylenchlorid zutropfen, rührt und kühlt auf 0° ab. Nach Beendigung der Zugabe rührt man das Reaktionsgemisch eine Stunde lang bei 0-5° und rührt anschliessend unter Rückflussbedingungen zwei Stunden lang. Nach Filtrieren erhält man das rohe 3-[5-(3-Chlorpropyl)-1H-imidazol-1-ylmethyl]-1,2-benzisoxazol mit einem Schmelzpunkt von 250-252°.

Beispiel 7:

a) 1,4 g (0,0056 Mol) 5-Chlor-3-[1-(1H-imidazol-1-yl)ethyl]-1,2-benzisoxazol werden in 100 ml Aceton gelöst und mit einer Lösung von 0,042 g (0,028 Mol) linksdrehender (-)-Weinsäure in 5 ml Aceton vermischt. Man lässt über Nacht bei Raumtemperatur stehen, trennt den Feststoff ab und rekristallisiert diesen zweimal aus absolutem Aethanol. Das Tartrat hat einen Schmelzpunkt von 149-151°. Dieses wird mit verdünnter wässriger Natriumhydroxid-Lösung in die freie Base umgewandelt. Anschliessend überführt man den biologisch aktiveren rechtsdrehenden Antipoden in das Hydrochlorid. Schmelzpunkt 169-170°; $[alpha]_D^{25}$ = + 5,56° (in Methanol).
b) Analog erhält man durch Umsetzen mit rechtsdrehender (+)-Weinsäure den linksdrehenden Antipoden. Schmelzpunkt Tartrat: 149-151°; Schmelzpunkt Hydrochlorid: 168-170°; $[alpha]_D^{25}$ = - 5,82° (in Methanol).

Beispiel 8:

a) 3-(1H-Imidazol-1-ylmethyl)-1,2-benzisothiazol
0,28 g (0,002 Mol) Natriumhydrid-Suspension in Mineralöl (50%ig) werden durch Auswaschen mit 3 x 5 ml n-Hexan von Mineralöl befreit und in 5 ml Dimethylformamid resuspendiert. Zu der gerührten Suspension werden 0,14 g (0,002 Mol) Imidazol bei Raumtemperatur in mehreren Portionen gegeben. Man lässt dreissig Minuten lang rühren und fügt 0,44 g (0,002 Mol) einer Lösung von 3-Brommethyl-1,2-benzisothiazol in 5 ml Dimethylformamid hinzu. Man erwärmt das Gemisch und lässt 20 Stunden lang bei 80-85° rühren. Nach Eindampfen nimmt man den Rückstand in einem Gemisch von 10 ml Wasser und Methylenchlorid auf, trennt die organische Phase ab, wäscht mit Wasser, trocknet über wasserfreiem Natriumsulfat und dampft ein. Man nimmt die rohe Titelverbindung als zähes Oel in 10 ml Aceton auf und überführt diese durch Behandeln mit einer chlorwasserstoffhaltigen Aetherlösung in das Hydrochlorid mit einem Schmelzpunkt von 224-226°.

Das Ausgangsmaterial 3-Brommethyl-1,2-benzisothiazol lässt sich gemäss H. Uno et al. Chem. Pharm. Bull. 26 (12), 3888 (1978) herstellen.

Beispiel 9:

a) Herstellung von 10.000 Tabletten mit 10,0 mg Wirkstoff:

| Bestandteile | |
|---|---|
| 5-Chlor-3-[1-(1H-imidazol-1-yl)ethyl]-1,2-benzisoxazol-hydrochlorid | 200,00 g |
| Lactose | 2.400,00 g |
| Maisstärke | 125,00 g |
| Polyethylenglycol 6,000 | 150,00 g |
| Magnesiumstearat | 40,00 g |
| Wasser (steril) | q.s. |

Das pulverige Material wird durch ein Sieb mit Oeffnungen von 0,6 mm Weite gedrückt. Anschliessend mischt man in einem Mischer den Wirkstoff mit der Lactose, dem Magnesiumstearat und der halben Menge Stärke. Die andere halbe Menge Stärke wird in 65 ml Wasser suspendiert und diese Suspension zu einer siedenden Lösung des Polyäthylenglycols 260 ml in Wasser gegeben. Diese gebildete Paste wird mit der Pulvermischung versetzt, welche gegebenenfalls mit weiterem Wasser granuliert wird. Man trocknet das Granulat bei 35°, treibt dieses durch ein Sieb mit 1,2 mm weiten Oeffnungen und komprimiert zu konkaven Tabletten mit oberer Bruchkerbe.
b) Herstellung von 1.000 Kapseln mit 10,0 mg Wirkstoff:

| Bestandteile | |
|---|---|
| 3,k-Dihydro-N,N-dipropyl-5-ethoxy-2H-[1]benzothiopyran-3-amin-hydrochlorid | 10,00 g |
| Lactose | 207,00 g |
| Stärke | 80,00 g |
| Magnesiumstearat | 3,00 g |

Das pulverige Material wird durch ein Sieb mit Oeffnungen von 0,6 mm Durchmesser gedrückt. Anschliessend mischt man in einem Mischer den Wirkstoff mit der Lactose, dem Magnesiumstearat und der Stärke bis man eine homogene Masse erhält. Man füllt Nr. 2 Hartgelatinekapseln mit 300 mg der vorbereiteten Mischung.

Analog kann man Kapseln mit 5 - 100 mg eines anderen von der Formel I definierten Wirkstoffs herstellen.

**Patentansprüche**

**Patentansprüche für folgende Vertragsstaaten : AT, BE, CH, DE, FR, GB, IT, LI, LU, NL, SE**

**1.** Verbindungen der Formel I

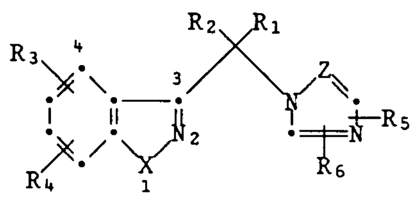

(I)

worin X Sauerstoff oder Schwefel, Z die Gruppe -CH= zur Bildung der Imidazol-1-yl-Gruppe oder ein Stickstoffheteroatom zur Bildung der 1,2,4-Triazol-1-yl-Gruppe, $R_1$ und $R_2$ unabhängig voneinander Wasserstoff, Niederalkyl, Niederalkenyl, Niederalkinyl oder Arylniederalkyl oder $R_1$ und $R_2$ zusammen Niederalkylen oder $R_1$ zusammen mit $R_5$ als Substituent des C-Atoms Z in der Imidazolylgruppe $C_2$-$C_4$-Alkylen, $R_3$ und $R_4$ unabhängig voneinander Wasserstoff, Niederalkyl, Cycloalkyl, Halogen, Trifluormethyl, Cyan, Nitro, Amino, Hydroxy, Niederalkanoyloxy, carbocyclisches Aroyloxy, Niederalkoxy oder carbocyclisches Aryl, $R_3$ und $R_4$ zusammen an nachbarständigen C-Atomen Niederalkylendioxy, $R_3$ und $R_4$ zusammen an nachbarständigen C-Atomen 1,3-Butadienylen oder $C_3$-$C_5$-Alkylen, wobei $R_3$ und $R_4$ mit diesen nachbarständigen C-Atomen eine entsprechende benzokondensierte Gruppe oder eine kondensierte $C_5$-$C_7$-Cycloalkenylgruppe ergibt, $R_5$ an einem Ring-C-Atom Wasserstoff, Niederalkyl oder Hydroxyniederalkyl, $R_6$ an einem Ring-C-Atom Wasserstoff oder Niederalkyl oder $R_5$ und $R_6$ zusammen $C_3$-$C_5$-Alkylen darstellen und dabei Z ein C-Atom darstellt und $R_5$ und $R_6$ zusammen dieses C-Atom und das nachbarständige C-Atom substituieren, und Salze dieser Verbindungen, wobei "nieder" bedeutet, dass die mit diesem Ausdruck definierten Gruppen oder Substituenten bis zu 7 C-Atome enthalten.

**2.** Verbindungen der Formel I gemäss Anspruch 1, worin X Schwefel und Z Kohlenstoff bedeutet.

**3.** Verbindungen der Formel I gemäss Anspruch 1, worin X Schwefel und Z Stickstoff bedeutet.

**4.** Verbindungen gemäss Anspruch 1 der Formel IIa

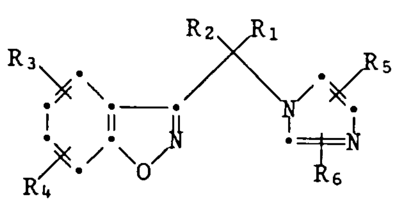

(IIa)

worin $R_1$ und $R_2$ unabhängig voneinander Wasserstoff, Niederalkyl, Arylniederalkyl, Niederalkenyl oder Niederalkinyl oder $R_1$ und $R_2$ zusammen Niederalkylen, $R_3$ und $R_4$ unabhängig voneinander Wasserstoff, Niederalkyl, Cycloalkyl, Halogen, Trifluormethyl, Cyan, Nitro, Amino, Hydroxy, Niederalkanoyloxy, carbocyclisches Aroyloxy, Niederalkoxy oder carbocyclisches Aryl oder $R_3$ und $R_4$ zusammen an nachbarständigen C-Atomen Niederalkylendioxy und $R_5$ und $R_6$ unabhängig voneinander Wasserstoff oder Niederalkyl bedeuten, und pharmazeutisch annehmbare Salze dieser Verbindungen, wobei "nieder" bedeutet, dass die mit diesem Ausdruck definierten Gruppen oder Substituenten bis zu 7 C-Atome enthalten.

**5.** Verbindungen gemäss Anspruch 4 der Formel IIa, worin $R_1$ Wasserstoff oder Niederalkyl, $R_2$ Wasserstoff oder Niederalkyl, $R_3$ Wasserstoff, Niederalkyl, $C_5$-$C_7$-Cycloalkyl, Halogen, Trifluormethyl, Cyan, Nitro, Amino, Hydroxy, Niederalkanoyloxy, carbocyclisches Aroyloxy, Niederalkoxy oder carbocyclisches Aryl, $R_4$ Wasserstoff, Niederalkyl, Niederalkoxy oder Halogen oder $R_3$ und $R_4$ zusammen an nachbarständigen C-Atomen Niederalkylendioxy und $R_5$ und $R_6$ unabhängig voneinander Wasserstoff oder Niederalkyl bedeuten, und pharmazeutisch annehmbare Salze davon, wobei "nieder" bedeutet, dass die mit diesem Ausdruck definierten Gruppen oder Substituenten bis zu 7 C-Atome enthalten.

**6.** Verbindungen gemäss Anspruch 4 der Formel IIa, worin $R_1$ Wasserstoff oder Niederalkyl, $R_2$ Wasserstoff oder Niederalkyl, $R_3$ Wasserstoff, Niederalkyl, Halogen, Trifluormethyl oder Phenyl, $R_4$ Wasserstoff, Niederalkyl oder Halogen bedeuten, $R_5$ sich in 4-Stellung des Imidazolylrings befindet und Wasserstoff oder Niederalkyl bedeutet, $R_6$ sich in 5-Stellung des Imidazolylrings befindet und Wasserstoff oder Niederalkyl bedeutet, und pharmazeutisch annehmbare Salze davon, wobei "nieder" bedeutet, dass die mit diesem Ausdruck definierten Gruppen oder Substituenten bis zu 7 C-Atome enthalten.

**7.** Verbindungen gemäss Anspruch 4 der Formel IIa, worin $R_1$ Wasserstoff oder Niederalkyl, $R_2$ Wasserstoff, $R_3$ Wasserstoff, Niederalkyl, Trifluormethyl, Halogen oder Phenyl und $R_4$, $R_5$ und $R_6$ Wasserstoff bedeuten, und pharmazeutisch annehmbare Salze davon, wobei "nieder" bedeutet, dass die mit diesem Ausdruck definierten Gruppen oder Substituenten bis zu 7 C-Atome enthalten.

**8.** Verbindungen gemäss Anspruch 1 der Formel IIb

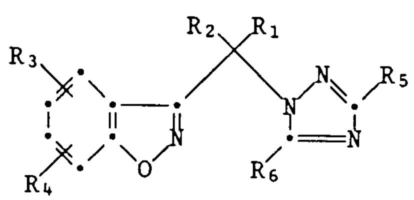

(IIb)

worin $R_1$ und $R_2$ unabhängig voneinander Wasserstoff, Niederalkyl, Arylniederalkyl, Niederalkenyl oder Niederalkinyl oder $R_1$ und $R_2$ zusammen Niederalkylen, $R_3$ und $R_4$ unabhängig voneinander Wasserstoff, Niederalkyl, Cycloalkyl, Halogen, Trifluormethyl, Cyan, Nitro, Amino, Hydroxy, Niederalkanoyloxy, carbocyclisches Aroyloxy, Niederalkoxy oder carbocyclisches Aryl oder $R_3$ und $R_4$ zusammen an nachbarständigen C-Atomen Niederalkylendioxy und $R_5$ und $R_6$ unabhängig voneinander Wasserstoff oder Niederalkyl bedeuten, und pharmazeutisch annehmbare Salze dieser Verbindungen, wobei "nieder" bedeutet, dass die mit diesem Ausdruck definierten Gruppen oder Substituenten bis zu 7 C-Atome enthalten.

**9.** 5-Chlor-3-[1-(1H-imidazol-1-yl)ethyl]-1,2-benzisoxazol gemäss Anspruch 1 und ein pharmazeutisch annehmbares Salz davon.

**10.** Der rechtsdrehende Antipode von 5-Chlor-3-[1-(1H-imidazol-1-yl)ethyl]-1,2-benzisoxazol und einen pharmazeutisch annehmbaren Salz davon.

**11.** Pharmazeutische Präparate enthaltend Verbindungen der Ansprüche 1-10 oder ein pharmazeutisch annehmbares Salz davon zusammen mit einem pharmazeutisch geeigneten Trägermaterial.

**12.** Die in den Ansprüchen 1 bis 10 genannten Verbindungen zur Anwendung in einem Verfahren zur

therapeutischen Behandlung des menschlichen oder tierischen Körpers.

**13.** Die in den Ansprüchen 1 bis 10 genannten Verbindungen zur Verwendung als Antiepileptika.

**14.** Verfahren zur Herstellung von den in Anspruch 1 genannten Verbindungen der Formel I, in der alle Symbole die in Anspruch 1 angegebenen Bedeutungen haben, und deren Salzen, dadurch gekennzeichnet, dass man die Kondensation einer Verbindung der Formel V

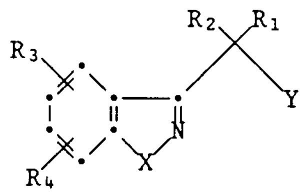

(V)

worin $R_1$-$R_4$ und X die genannten Bedeutungen haben, und Y eine reaktionsfähige, veresterte Hydroxygruppe darstellt, mit einer Verbindung der Formel VIa oder VIb

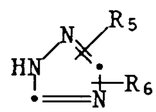

(VIa)

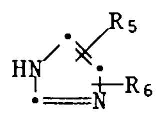

(VIb)

worin $R_5$ an einem Ring-C-Atom Wasserstoff, Niederalkyl oder Hydroxyniederalkyl, vorzugsweise in geschützter Form und $R_6$ an einem Ring-C-Atom Wasserstoff oder Niederalkyl bedeutet, oder worin $R_5$ und $R_6$ zusammen $C_3$-$C_5$-Alkylen darstellen und dabei Z ein C-Atom darstellt und $R_5$ und $R_6$ zusammen dieses C-Atom und das nachbarständige C-Atom substituieren, ausführt und gewünschtenfalls reaktive funktionelle Gruppen intermediär bei der Ausführung eines der genannten Verfahren schützt und anschliessend die freie Verbindung der Formel I isoliert und/oder eine erhältliche Verbindung der Formel I in eine andere Verbindung der Formel I umwandelt und/oder eine erhältliche freie Verbindung in ein Salz oder ein Salz in die freie Verbindung oder in ein anderes Salz umwandelt und/oder ein erhältliches Gemisch von Isomeren oder Racematen in die einzelnen Isomere oder Racematen auftrennt und/oder ein Racemat in die optischen Antipoden aufspaltet, wobei "nieder" bedeutet, dass die mit diesem Ausdruck definierten Gruppen oder Substituenten bis zu 7 C-Atome enthalten.

**15.** Verfahren zur Herstellung einer Verbindung der im Anspruch 1 genannten Formel I, worin Z die Gruppe -CH= darstellt und die übrigen Symbole die im Anspruch 1 genannten Bedeutungen haben, und deren Salzen, dadurch gekennzeichnet, dass man eine Verbindung der Formel (V) (wie im Anspruch 14 definiert) mit einer Verbindung der Formel VII

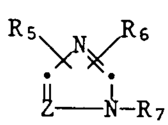

(VII)

worin $R_5$ Wasserstoff, Niederalkyl oder Hydroxyniederalkyl, $R_6$ Wasserstoff oder Niederalkyl oder $R_5$ und $R_6$ zusammen $C_3$-$C_5$-Alkylen darstellen und dabei Z ein C-Atom darstellt und $R_5$ und $R_6$ zusammen dieses C-Atom und das nachbarständige C-Atom substituieren, und $R_7$ eine übliche Aminoschutzgruppe darstellt, kondensiert und anschliessend die Aminoschutzgruppe abspaltet, vorzugsweise in-situ, und gewünschtenfalls reaktive funktionelle Gruppen intermediär bei der Ausführung eines der genannten Verfahren schützt und anschliessend die freie Verbindung der Formel I isoliert und/oder eine erhältliche Verbindung der Formel I in eine andere Verbindung der Formel I umwandelt

und/oder eine erhältliche freie Verbindung in ein Salz oder ein Salz in die freie Verbindung oder in ein anderes Salz umwandelt und/oder ein erhältliches Gemisch von Isomeren oder Racematen in die einzelnen Isomere oder Racematen auftrennt und/oder ein Racemat in die optischen Antipoden aufspaltet, wobei "nieder" bedeutet, dass die mit diesem Ausdruck definierten Gruppen oder Substituenten bis zu 7 C-Atome enthalten.

**16.** Verfahren zur Herstellung von Verbindungen der in Anspruch 1 genannten Formel I, worin Z die Gruppe =CH-darstellt und $R_1$ zusammen mit $R_5$ als Substituent des C-Atoms in der Imidazolylgruppe $C_2$-$C_4$-Alkylen bedeuten, und deren Salzen, dadurch gekennzeichnet, dass man eine Verbindung der Formel VIII

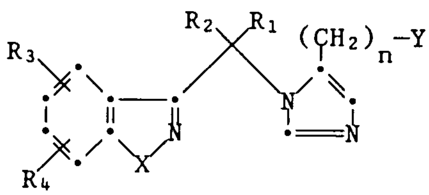

(VIII)

worin X, $R_3$ und $R_4$ wie in Anspruch 1 definiert sind, $R_2$ die in Anspruch 1 genannten Bedeutungen, ausser Niederalkylen gemeinsam mit $R_1$, hat und Y reaktives verestertes Hydroxy bedeutet, in Gegenwart einer Base zyklisiert und gewünschtenfalls reaktive funktionelle Gruppen intermediär bei der Ausführung eines der genannten Verfahren schützt und anschliessend die freie Verbindung der Formel I isoliert und/oder eine erhältliche Verbindung der Formel I in eine andere Verbindung der Formel I umwandelt und/oder eine erhältliche freie Verbindung in ein Salz oder ein Salz in die freie Verbindung oder in ein anderes Salz umwandelt und/oder ein erhältliches Gemisch von Isomeren oder Racematen in die einzelnen Isomere oder Racematen auftrennt und/oder ein Racemat in die optischen Antipoden aufspaltet, wobei "nieder" bedeutet, dass die mit diesem Ausdruck definierten Gruppen oder Substituenten bis zu 7 C-Atome enthalten.

**Patentansprüche für folgende Vertragsstaaten : ES, GR**

**1.** Verfahren zur Herstellung von Verbindungen der Formel I

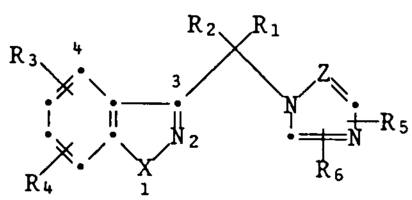

(I)

worin X Sauerstoff oder Schwefel, Z die Gruppe -CH= zur Bildung der Imidazol-1-yl-Gruppe oder ein Stickstoffheteroatom zur Bildung der 1,2,4-Triazol-1-yl-Gruppe, $R_1$ und $R_2$ unabhängig voneinander Wasserstoff, Niederalkyl, Niederalkenyl mit 3-7 Kohlenstoffatomen, Niederalkinyl mit 3-7 Kohlenstoffatomen oder Arylniederalkyl oder $R_1$ und $R_2$ zusammen Niederalkylen oder $R_1$ zusammen mit $R_5$ als Substituent des C-Atoms Z in der Imidazolylgruppe $C_2$-$C_4$-Alkylen, $R_3$ und $R_4$ unabhängig voneinander Wasserstoff, Niederalkyl, Cycloalkyl, Halogen, Trifluormethyl, Cyan, Nitro, Amino, Hydroxy, Niederalkanoyloxy, carbocyclisches Aroyloxy, Niederalkoxy oder carbocyclisches Aryl, $R_3$ und $R_4$ zusammen an nachbarständigen C-Atomen Niederalkylendioxy, $R_3$ und $R_4$ zusammen an nachbarständigen C-Atomen 1,3-Butadienylen oder $C_3$-$C_5$-Alkylen, wobei $R_3$ und $R_4$ mit diesen nachbarständigen C-Atomen eine entsprechende benzokondensierte Gruppe oder eine kondensierte $C_5$-$C_7$-Cycloalkenylgruppe ergibt, $R_5$ an einem Ring-C-Atom Wasserstoff, Niederalkyl oder Hydroxyniederalkyl, $R_6$ an einem Ring-C-Atom Wasserstoff oder Niederalkyl oder $R_5$ und $R_6$ zusammen $C_3$-$C_5$-Alkylen darstellen und dabei Z ein C-Atom darstellt und $R_5$ und $R_6$ zusammen dieses C-Atom und das nachbarständige C-Atom substituieren, und Salze dieser Verbindungen, dadurch gekennzeichnet, dass man die Kondensation einer Verbindung der Formel V

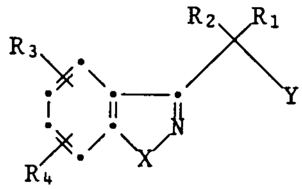

(V)

worin $R_1$-$R_4$ und X die genannten Bedeutungen haben, und Y eine reaktionsfähige, veresterte Hydroxygruppe darstellt, mit einer Verbindung der Formel VIa oder VIb

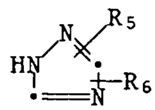

(VIa)

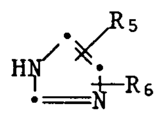

(VIb)

worin $R_5$ an einem Ring-C-Atom Wasserstoff, Niederalkyl oder Hydroxyniederalkyl, vorzugsweise in geschützter Form und $R_6$ an einem Ring-C-Atom Wasserstoff oder Niederalkyl bedeutet, oder worin $R_5$ und $R_6$ zusammen $C_3$-$C_5$-Alkylen darstellen und dabei Z ein C-Atom darstellt und $R_5$ und $R_6$ zusammen dieses C-Atom und das nachbarständige C-Atom substituieren, ausführt und gewünschtenfalls reaktive funktionelle Gruppen intermediär bei der Ausführung eines der genannten Verfahren schützt und anschliessend die freie Verbindung der Formel I isoliert und/oder eine erhältliche Verbindung der Formel I in eine andere Verbindung der Formel I umwandelt und/oder eine erhältliche freie Verbindung in ein Salz oder ein Salz in die freie Verbindung oder in ein anderes Salz umwandelt und/oder ein erhältliches Gemisch von Isomeren oder Racematen in die einzelnen Isomere oder Racematen auftrennt und/oder ein Racemat in die optischen Antipoden aufspaltet, wobei "nieder" bedeutet, dass die mit diesem Ausdruck definierten Gruppen oder Substituenten bis zu 7 C-Atome enthalten.

**2.** Verfahren zur Herstellung einer Verbindung der im Anspruch 1 genannten Formel I, worin Z die Gruppe -CH= darstellt und die übrigen Symbole die im Anspruch 1 genannten Bedeutungen haben, und deren Salzen, dadurch gekennzeichnet, dass man eine Verbindung der Formel V (wie im Anspruch 14 definiert) mit einer Verbindung der Formel VII

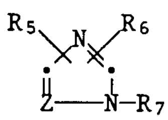

(VII)

worin $R_5$ Wasserstoff, Niederalkyl oder Hydroxyniederalkyl, $R_6$ Wasserstoff oder Niederalkyl oder $R_5$ und $R_6$ zusammen $C_3$-$C_5$-Alkylen darstellen und dabei Z ein C-Atom darstellt und $R_5$ und $R_6$ zusammen dieses C-Atom und das nachbarständige C-Atom substituieren, und $R_7$ eine übliche Aminoschutzgruppe darstellt, kondensiert und anschliessend die Aninoschutzgruppe abspaltet, vorzugsweise in-situ, und gewünschtenfalls reaktive funktionelle Gruppen intermediär bei der Ausführung eines der genannten Verfahren schützt und anschliessend die freie Verbindung der Formel I isoliert und/oder eine erhältliche Verbindung der Formel I in eine andere Verbindung der Formel I umwandelt und/oder eine erhältliche freie Verbindung in ein Salz oder ein Salz in die freie Verbindung oder in ein anderes Salz umwandelt und/oder ein erhältliches Gemisch von Isomeren oder Racematen in die einzelnen Isomere oder Racematen auftrennt und/oder ein Racemat in die optischen Antipoden aufspaltet, wobei "nieder" bedeutet, dass die mit diesem Ausdruck definierten Gruppen oder Substituenten bis zu 7 C-Atome enthalten.

**3.** Verfahren zur Herstellung von Verbindungen der in Anspruch 1 genannten Formel I, worin Z die Gruppe =CH-darstellt und $R_1$ zusammen mit $R_5$ als Substituent des C-Atoms in der Imidazolylgruppe $C_2$-$C_4$-

Alkylen bedeuten, und deren Salzen, dadurch gekennzeichnet, dass man eine Verbindung der Formel VIII

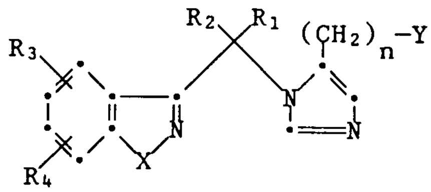

(VIII)

worin X, $R_3$ und $R_4$ wie in Anspruch 1 weiter vorn definiert sind, $R_2$ die in Anspruch 1 genannten Bedeutungen, ausser Niederalkylen gemeinsam mit $R_1$, hat und Y reaktives verestertes Hydroxy bedeutet, in Gegenwart einer Base zyklisiert und gewünschtenfalls reaktive funktionelle Gruppen intermediär bei der Ausführung eines der genannten Verfahren schützt und anschliessend die freie Verbindung der Formel I isoliert und/oder eine erhältliche Verbindung der Formel I in eine andere Verbindung der Formel I umwandelt und/oder eine erhältliche freie Verbindung in ein Salz oder ein Salz in die freie Verbindung oder in ein anderes Salz umwandelt und/oder ein erhältliches Gemisch von Isomeren oder Racematen in die einzelnen Isomere oder Racematen auftrennt und/oder ein Racemat in die optischen Antipoden aufspaltet, wobei "nieder" bedeutet, dass die mit diesem Ausdruck definierten Gruppen oder Substituenten bis zu 7 C-Atome enthalten.

**4.** Verfahren gemäss einem der Ansprüche 1, 2 oder 3 zur Herstellung von Verbindungen der Formel I, worin X Schwefel und Z Kohlenstoff bedeutet.

**5.** Verfahren gemäss einem der Ansprüche 1, 2 oder 3 Zur Herstellung von Verbindungen der Formel I, worin X Schwefel und Z Stickstoff bedeutet.

**6.** Verfahren gemäss einem der Ansprüche 1 oder 2 zur Herstellung von Verbindungen der Formel IIa

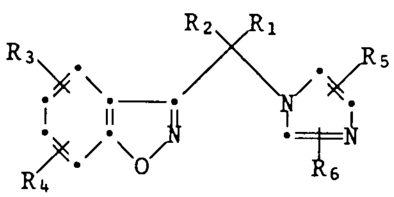

(IIa)

worin $R_1$ und $R_2$ unabhängig voneinander Wasserstoff, Niederalkyl, Arylniederalkyl, Niederalkenyl oder Niederalkinyl oder $R_1$ und $R_2$ zusammen Niederalkylen, $R_3$ und $R_4$ unabhängig voneinander Wasserstoff, Niederalkyl, Cycloalkyl, Halogen, Trifluormethyl, Cyan, Nitro, Amino, Hydroxy, Niederalkanoyloxy, carbocyclisches Aroyloxy, Niederalkoxy oder carbocyclisches Aryl oder $R_3$ und $R_4$ zusammen an nachbarständigen C-Atomen Niederalkylendioxy und $R_5$ und $R_6$ unabhängig voneinander Wasserstoff oder Niederalkyl bedeuten, und pharmazeutisch annehmbare Salze dieser Verbindungen, wobei "nieder" bedeutet, dass die mit diesem Ausdruck definierten Gruppen oder Substituenten bis zu 7 C-Atome enthalten.

**7.** Verfahren gemäss Anspruch 6 zur Herstellung von Verbindungen der Formel IIa, worin $R_1$ Wasserstoff oder Niederalkyl, $R_2$ Wasserstoff oder Niederalkyl, $R_3$ Wasserstoff, Niederalkyl, $C_5$-$C_7$-Cycloalkyl, Halogen, Trifluormethyl, Cyan, Nitro, Anino, Hydroxy, Niederalkanoyloxy, carbocyclisches Aroyloxy, Niederalkoxy oder carbocyclisches Aryl, $R_4$ Wasserstoff, Niederalkyl, Niederalkoxy oder Halogen oder $R_3$ und $R_4$ zusammen an nachbarständigen C-Atomen Niederalkyl bedeuten, und pharmazeutisch annehmbare Salze davon, wobei "nieder" bedeutet, dass die mit diesem Ausdruck definierten Gruppen oder Substituenten bis zu 7 C-Atome enthalten.

**8.** Verfahren gemäss Anspruch 6 zur Herstellung von Verbindungen der Formel IIa, worin $R_1$ Wasserstoff oder Niederalkyl, $R_2$ Wasserstoff oder Niederalkyl, $R_3$ Wasserstoff, Niederalkyl, Halogen, Trifluormethyl oder Phenyl, $R_4$ Wasserstoff, Niederalkyl oder Halogen bedeuten, $R_5$ sich in 4-Stellung des Imidazolylrings befindet und Wasserstoff oder Niederalkyl bedeutet, $R_6$ sich in 5-Stellung des Imidazolylrings befindet und Wasserstoff oder Niederalkyl bedeutet, und pharmazeutisch annehmbare Salze davon, wobei "nieder" bedeutet, dass die mit diesem Ausdruck definierten Gruppen oder Substituenten bis zu 7 C-Atome enthalten.

**9.** Verfahren gemäss Anspruch 6 zur Herstellung von Verbindungen der Formel IIa, worin $R_1$ Wasserstoff oder Niederalkyl, $R_2$ Wasserstoff, $R_3$ Wasserstoff, Niederalkyl, Trifluormethyl, Halogen oder Phenyl und $R_4$, $R_5$ und $R_6$ Wasserstoff bedeuten, und pharmazeutisch annehmbare Salze davon, wobei "nieder" bedeutet, dass die mit diesem Ausdruck definierten Gruppen oder Substituenten bis zu 7 C-Atome enthalten.

**10.** Verfahren gemäss einem der Ansprüche 1 bis 3 zur Herstellung von Verbindungen der Formel IIb

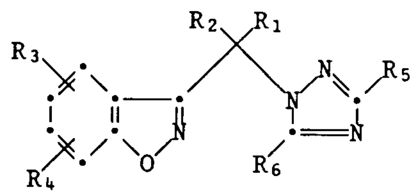

(IIb)

worin $R_1$ und $R_2$ unabhängig voneinander Wasserstoff, Niederalkyl, Arylniederalkyl, Niederalkenyl oder Niederalkinyl oder $R_1$ und $R_2$ zusammen Niederalkylen, $R_3$ und $R_4$ unabhängig voneinander Wasserstoff, Niederalkyl, Cycloalkyl, Halogen, Trifluormethyl, Cyan, Nitro, Amino, Hydroxy, Niederalkanoyloxy, carbocyclisches Aroyloxy, Niederalkoxy oder carbocyclisches Aryl oder $R_3$ und $R_4$ zusammen an nachbarständigen C-Atomen Niederalkylendioxy und $R_5$ und $R_6$ unabhängig voneinander Wasserstoff oder Niederalkyl bedeuten, und pharmazeutisch annehmbare Salze dieser Verbindungen, wobei "nieder" bedeutet, dass die mit diesem Ausdruck definierten Gruppen oder Substituenten bis zu 7 C-Atome enthalten.

**11.** Verfahren gemäss Anspruch 1 zur Herstellung von 5-Chlor-3-[1-(1H-imidazol-1-yl)ethyl]-1,2-benzisoxazol und einem pharmazeutisch annehmbaren Salz davon.

**12.** Verfahren gemäss Anspruch 1 zur Herstellung des rechtsdrehenden Antipoden von 5-Chlor-3-[1-(1H-imidazol-1-yl)ethyl]-1,2-benzisoxzol und eines pharmazeutisch annehmbaren Salzes davon.

## Claims

**Claims for the following Contracting States : AT, BE, CH, DE, FR, GB, IT, LI, LU, NL, SE**

**1.** A componnd of formula I

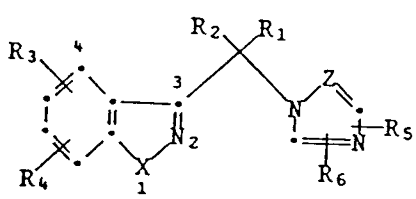

(I)

wherein X represents oxygen or sulfur, Z represents the group -CH= so as to complete an imidazol-1-yl group or Z represents a nitrogen hetero atom so as to complete a 1,2,4-triazol-1-yl group, $R_1$ and $R_2$ independently represent hydrogen, lower alkyl, lower alkenyl, lower alkynyl or aryl-lower alkyl, or $R_1$ and $R_2$ together represent lower alkylene, or $R_1$ together with $R_5$ as substituent of the Z-carbon atom in the imidazolyl group represent $C_2$-$C_4$alkylene, $R_3$ and $R_4$ independently represent hydrogen, lower alkyl, cycloalkyl, halogen, trifluoromethyl, cyano, nitro, amino, hydroxy, lower alkanoyloxy, carbocyclic aroyloxy, lower alkoxy or carbocyclic aryl, or $R_3$ and $R_4$ together, when located on adjacent carbon atoms, represent lower alkylenedioxy, or $R_3$ and $R_4$ together, when located on adjacent carbon atoms, represent 1,3-butadienylene or $C_3$-$C_5$alkylene, $R_3$ and $R_4$ forming with those adjacent carbon atoms a

benzo-fused group or a fused $C_5$-$C_7$cycloalkenyl group, respectively, $R_5$ located on a ring carbon atom represents hydrogen, lower alkyl or hydroxy-lower alkyl, $R_6$ located on a ring carbon atom represents hydrogen or lower alkyl, or $R_5$ and $R_6$ together represent $C_3$-$C_5$alkylene and, at the same time, Z represents a carbon atom, and $R_5$ and $R_6$ together substitute that carbon atom and the adjacent carbon atom, or a salt of that compound, "lower" denoting that the groups or substituents so defined contain up to 7 carbon atoms.

**2.** A compound of formula I according to claim 1 wherein X represents sulfur and Z represents carbon.

**3.** A compound of formula I according to claim 1 wherein X represents sulfur and Z represents nitrogen.

**4.** A compound according to claim 1 of formula IIa

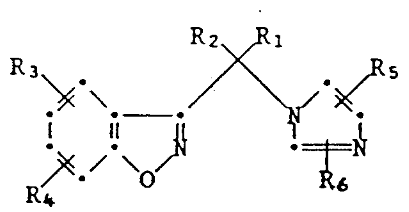

(IIa)

wherein $R_1$ and $R_2$ independently represent hydrogen, lower alkyl, aryl-lower alkyl, lower alkenyl or lower alkynyl, or $R_1$ and $R_2$ together represent lower alkylene, $R_3$ and $R_4$ independently represent hydrogen, lower alkyl, cycloalkyl, halogen, trifluoromethyl, cyano, nitro, amino, hydroxy, lower alkanoyloxy, carbocyclic aroyloxy, lower alkoxy or carbocyclic aryl, or $R_3$ and $R_4$ together, when located on adjacent carbon atoms, represent lower alkylenedioxy, and $R_5$ and $R_6$ independently represent hydrogen or lower alkyl, or a pharmaceutically acceptable salt of that compound, "lower" denoting that the groups or substituents so defined contain up to 7 carbon atoms.

**5.** A compound according to claim 4 of formula IIa wherein $R_1$ represents hydrogen or lower alkyl, $R_2$ represents hydrogen or lower alkyl, $R_3$ represents hydrogen, lower alkyl, $C_5$-$C_7$cycloalkyl, halogen, trifluoromethyl, cyano, nitro, amino, hydroxy, lower alkanoyloxy, carbocyclic aroyloxy, lower alkoxy or carbocyclic aryl, $R_4$ represents hydrogen, lower alkyl, lower alkoxy or halogen, or $R_3$ and $R_4$ together, when located on adjacent carbon atoms, represent lower alkylenedioxy, and $R_5$ and $R_6$ independently represent hydrogen or lower alkyl, or a pharmaceutically acceptable salt thereof, "lower" denoting that the groups or substituents so defined contain up to 7 carbon atoms.

**6.** A compound according to claim 4 of formula IIa wherein $R_1$ represents hydrogen or lower alkyl, $R_2$ represents hydrogen or lower alkyl, $R_3$ represents hydrogen, lower alkyl, halogen, trifluoromethyl or phenyl, $R_4$ represents hydrogen, lower alkyl or halogen, $R_5$ is located at the 4-position of the imidazolyl ring and represents hydrogen or lower alkyl, $R_6$ is located at the 5-position of the imidazolyl ring and represents hydrogen or lower alkyl, or a pharmaceutically acceptable salt thereof, "lower" denoting that the groups or substituents so defined contain up to 7 carbon atoms.

**7.** A compound according to claim 4 of formula IIa wherein $R_1$ represents hydrogen or lower alkyl, $R_2$ represents hydrogen, $R_3$ represents hydrogen, lower alkyl, trifluoromethyl, halogen or phenyl, and $R_4$, $R_5$ and $R_6$ representhydrogen, or a pharmaceutically acceptable salt thereof, "lower" denoting that the groups or substituents so defined contain up to 7 carbon atoms.

**8.** A compound according to claim 1 of formula IIb

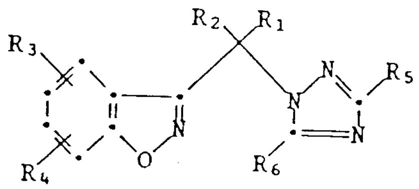

(IIb)

wherein $R_1$ and $R_2$ independently represent hydrogen, lower alkyl, aryl-lower alkyl, lower alkenyl or lower alkynyl, or $R_1$ and $R_2$ together represent lower alkylene, $R_3$ and $R_4$ independently represent hydrogen, lower alkyl, cycloalkyl, halogen, trifluoromethyl, cyano, nitro, amino, hydroxy, lower alkanoyloxy, carbocyclic aroyloxy, lower alkoxy or carbocyclic aryl, or $R_3$ and $R_4$ together, when located on adjacent carbon atoms, represent lower alkylenedioxy, and $R_5$ and $R_6$ independently represent hydrogen or lower alkyl, or a pharmaceutically acceptable salt of that compound, "lower" denoting that the groups or substituents so defined contain up to 7 carbon atoms.

**9.** 5-Chloro-3-[1-(1H-imidazol-1-yl)ethyl]-1,2-benzisoxazole according to claim 1 or a pharmaceutically acceptable salt thereof.

**10.** The dextrorotatory antipode of 5-chloro-3-[1-(1H-imidazol-1-yl)ethyl]-1,2-benzisoxazole or a pharmaceutically acceptable salt thereof.

**11.** A pharmaceutical composition comprising any one of the compounds of claims 1 to 10 or a pharmaceutically acceptable salt thereof together with a pharmaceutically acceptable carrier.

**12.** Any one of the compounds mentioned in claims 1 to 10 for use in a method for the therapeutic treatment of the human or animal body.

**13.** Any one of the compounds mentioned in claims 1 to 10 for use as an anti-epileptic.

**14.** A process for the preparation of a compound of formula I mentioned in claim 1, wherein all of the symbols are as defined in claim 1, or a salt thereof, in which process the condensation of a compound of formula V

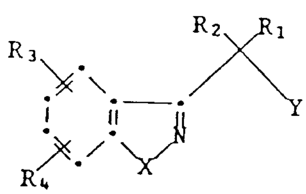

(V),

wherein $R_1$ to $R_4$ and X are as defined, and Y represents a reactive esterified hydroxy group, is carried out with a compound of formula VIa or VIb

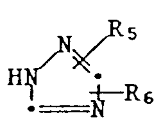

(VIa)

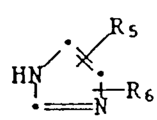

(VIb)

wherein $R_5$ located on a ring carbon atom represents hydrogen, lower alkyl or hydroxy-lower alkyl, preferably in protected form, and $R_6$ located on a ring carbon atom represents hydrogen or lower alkyl, or wherein $R_5$ and $R_6$ together represent $C_3$-$C_5$ alkylene and, at the same time, Z represents a carbon atom, and $R_5$ and $R_6$ together substitute that carbon atom and the adjacent carbon atom, and, if desired, reactive functional groups are protected intermediately during the performance of one of the

processes mentioned and then the free compound of formula I is isolated and/or an obtainable compound of formula I is converted into a different compound of formula I and/or an obtainable free compound is converted into a salt or a salt is converted into the free compound or into a different salt and/or an obtainable mixture of isomers or racemates is separated into the individual isomers or racemates and/or a racemate is resolved into the optical antipodes, "lower" denoting that the groups or substituents so defined contain up to 7 carbon atoms.

**15.** A process for the preparation of a compound of the formula I mentioned in claim 1, wherein Z represents the group -CH= and the other symbols are as defined in claim 1, or a salt thereof, in which process a compound of formula V (as defined in claim 14) is condensed with a compound of formula VII

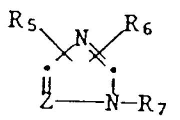

(VII)

wherein $R_5$ represents hydrogen, lower alkyl or hydroxy-lower alkyl, $R_6$ represents hydrogen or lower alkyl, or $R_5$ and $R_6$ together represent $C_3$-$C_5$alkylene and, at the same time, Z represents a carbon atom, and $R_5$ and $R_6$ together substitute that carbon atom and the adjacent carbon atom, and $R_7$ represents a customary amino-protecting group, and then the amino-protecting group is removed, preferably in situ, and, if desired, reactive functional groups are protected intermediately during the performance of one of the processes mentioned and then the free compound of formula I is isolated and/or an obtainable compound of formula I is converted into a different compound of formula I and/or an obtainable free compound is converted into a salt or a salt is converted into the free compound or into a different salt and/or an obtainable mixture of isomers or racemates is separated into the individual isomers or racemates and/or a racemate is resolved into the optical antipodes, "lower" denoting that the groups or substituents so defined contain up to 7 carbon atoms.

**16.** A process for the preparation of a compound of the formula I mentioned in claim 1, wherein Z represents the group -CH= and $R_1$ together with $R_5$ as substituent of the carbon atom in the imidazolyl group represent $C_2$-$C_4$alkylene, or a salt thereof, in which process a compound of formula VIII

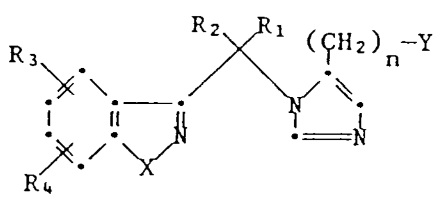

(VIII)

wherein X, $R_3$ and $R_4$ are as defined in claim 1, $R_2$ is as defined in claim 1, except that it is not lower alkylene together with $R_1$, and Y represents reactive esterified hydroxy, is cyclised in the presence of a base and, if desired, reactive functional groups are protected intermediately during the performance of one of the processes mentioned and then the free compound of formula I is isolated and/or an obtainable compound of formula I is converted into a different compound of formula I and/or an obtainable free compound is converted into a salt or a salt is converted into the free compound or into a different salt and/or an obtainable mixture of isomers or racemates is separated into the individual isomers or racemates and/or a racemate is resolved into the optical antipodes, "lower" denoting that the groups or substituents so defined contain up to 7 carbon atoms.

**Claims for the following Contracting States : ES, GR**

**1.** A process for the preparation of a compound of formula I

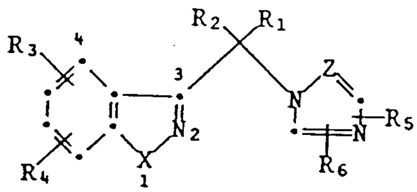

(I)

wherein X represents oxygen or sulfur, Z represents the group -CH = so as to complete an imidazol-1-yl group or Z represents a nitrogen hetero atom so as to complete a 1,2,4-triazol-1-yl group, $R_1$ and $R_2$ independently represent hydrogen, lower alkyl, lower alkenyl having from 3 to 7 carbon atoms, lower alkynyl having from 3 to 7 carbon atoms, or aryl-lower alkyl, or $R_1$ and $R_2$ together represent lower alkylene, or $R_1$ together with $R_5$ as substituent of the Z-carbon atom in the imidazolyl group represent $C_2$-$C_4$ alkylene, $R_3$ and $R_4$ independently represent hydrogen, lower alkyl, cycloalkyl, halogen, trifluoromethyl, cyano, nitro, amino, hydroxy, lower alkanoyloxy, carbocyclic aroyloxy, lower alkoxy or carbocyclic aryl, or $R_3$ and $R_4$ together, when located on adjacent carbon atoms, represent lower alkylenedioxy, or $R_3$ and $R_4$ together, when located on adjacent carbon atoms, represent 1,3-butadienylene or $C_3$-$C_5$ alkylene, $R_3$ and $R_4$ forming with those adjacent carbon atoms a benzo-fused group or a fused $C_5$-$C_7$ cycloalkenyl group, respectively, $R_5$ located on a ring carbon atom represents hydrogen, lower alkyl or hydroxy-lower alkyl, $R_6$ located on a ring carbon atom represents hydrogen or lower alkyl, or $R_5$ and $R_6$ together represent $C_3$-$C_5$ alkylene and, at the same time, Z represents a carbon atom, and $R_5$ and $R_6$ together substitute that carbon atom and the adjacent carbon atom, or a salt of that compound, in which process the condensation of a compound of formula V

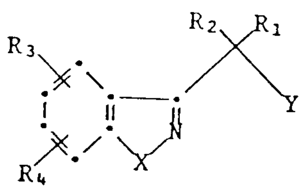

(V),

wherein $R_1$ to $R_4$ and X are as defined, and Y represents a reactive esterified hydroxy group, is carried out with a compound of formula VIa or VIb

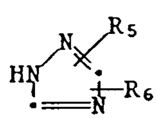

(VIa)

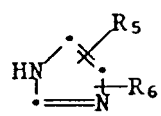

(VIb)

wherein $R_5$ located on a ring carbon atom represents hydrogen, lower alkyl or hydroxy-lower alkyl, preferably in protected form, and $R_6$ located on a ring carbon atom represents hydrogen or lower alkyl, or wherein $R_5$ and $R_6$ together represent $C_3$-$C_5$ alkylene and, at the same time, Z represents a carbon atom, and $R_5$ and $R_6$ together substitute that carbon atom and the adjacent carbon atom, and, if desired, reactive functional groups are protected intermediately during the performance of one of the processes mentioned and then the free compound of formula I is isolated and/or an obtainable compound of formula I is converted into a different compound of formula I and/or an obtainable free compound is converted into a salt or a salt is converted into the free compound or into a different salt and/or an obtainable mixture of isomers or racemates is separated into the individual isomers or racemates and/or a racemate is resolved into the optical antipodes, "lower" denoting that the groups or substituents so defined contain up to 7 carbon atoms.

**2.** A process for the preparation of a compound of the formula I mentioned in claim 1, wherein Z represents the group -CH = and the other symbols are as defined in claim 1, or a salt thereof, in which process a compound of formula V (as defined in claim 14) is condensed with a compound of formula

VII

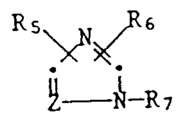

(VII)

wherein $R_5$ represents hydrogen, lower alkyl or hydroxy-lower alkyl, $R_6$ represents hydrogen or lower alkyl, or $R_5$ and $R_6$ together represent $C_3$-$C_5$alkylene and, at the same time, Z represents a carbon atom, and $R_5$ and $R_6$ together substitute that carbon atom and the adjacent carbon atom, and $R_7$ represents a customary amino-protecting group, and then the amino-protecting group is removed, preferably in situ, and, if desired, reactive functional groups are protected intermediately during the performance of one of the processes mentioned and then the free compound of formula I is isolated and/or an obtainable compound of formula I is converted into a different compound of formula I and/or an obtainable free compound is converted into a salt or a salt is converted into the free compound or into a different salt and/or an obtainable mixture of isomers or racemates is separated into the individual isomers or racemates and/or a racemate is resolved into the optical antipodes, "lower" denoting that the groups or substituents so defined contain up to 7 carbon atoms.

**3.** A process for the preparation of a compound of the formula I mentioned in claim 1, wherein Z represents the group -CH= and $R_1$ together with $R_5$ as substituent of the carbon atom in the imidazolyl group represent $C_2$-$C_4$alkylene, or a salt thereof, in which process a compound of formula VIII

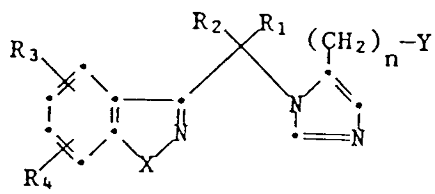

(VIII)

wherein X, $R_3$ and $R_4$ are as defined in claim 1 hereinbefore, $R_2$ is as defined in claim 1, except that it is not lower alkylene together with $R_1$, and Y represents reactive esterified hydroxy, is cyclised in the presence of a base and, if desired, reactive functional groups are protected intermediately during the performance of one of the processes mentioned and then the free compound of formula I is isolated and/or an obtainable compound of formula I is converted into a different compound of formula I and/or an obtainable free compound is converted into a salt or a salt is converted into the free compound or into a different salt and/or an obtainable mixture of isomers or racemates is separated into the individual isomers or racemates and/or a racemate is resolved into the optical antipodes, "lower" denoting that the groups or substituents so defined contain up to 7 carbon atoms.

**4.** A process according to any one of claims 1, 2 and 3 for the preparation of a compound of formula I wherein X represents sulfur and Z represents carbon.

**5.** A process according to any one of claims 1, 2 and 3 for the preparation of a compound of formula I wherein X represents sulfur and Z represents nitrogen.

**6.** A process according to either claim 1 or claim 2 for the preparation of a compound of formula IIa

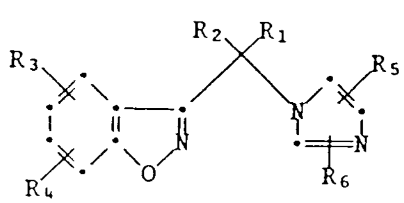

(IIa)

wherein $R_1$ and $R_2$ independently represent hydrogen, lower alkyl, aryl-lower alkyl, lower alkenyl or lower alkynyl, or $R_1$ and $R_2$ together represent lower alkylene, $R_3$ and $R_4$ independently represent hydrogen, lower alkyl, cycloalkyl, halogen, trifluoromethyl, cyano, nitro, amino, hydroxy, lower alkanoyloxy, carbocyclic aroyloxy, lower alkoxy or carbocyclic aryl, or $R_3$ and $R_4$ together, when located on adjacent carbon atoms, represent lower alkylenedioxy, and $R_5$ and $R_6$ independently represent hydrogen or lower alkyl, or a pharmaceutically acceptable salt of that compound, "lower" denoting that the groups or substituents so defined contain up to 7 carbon atoms.

**7.** A process according to claim 6 for the preparation of a compound of formula IIa wherein $R_1$ represents hydrogen or lower alkyl, $R_2$ represents hydrogen or lower alkyl, $R_3$ represents hydrogen, lower alkyl, $C_5$-$C_7$cycloalkyl, halogen, trifluoromethyl, cyano, nitro, amino, hydroxy, lower alkanoyloxy, carbocyclic aroyloxy, lower alkoxy or carbocyclic aryl, $R_4$ represents hydrogen, lower alkyl, lower alkoxy or halogen, or $R_3$ and $R_4$ together, when located on adjacent carbon atoms, represent lower alkyl, or a pharmaceutically acceptable salt thereof, "lower" denoting that the groups or substituents so defined contain up to 7 carbon atoms.

**8.** A process according to claim 6 for the preparation of a compound of formula IIa wherein $R_1$ represents hydrogen or lower alkyl, $R_2$ represents hydrogen or lower alkyl, $R_3$ represents hydrogen, lower alkyl, halogen, trifluoromethyl or phenyl, $R_4$ represents hydrogen, lower alkyl or halogen, $R_5$ is located at the 4-position of the imidazolyl ring and represents hydrogen or lower alkyl, $R_6$ is located at the 5-position of the imidazolyl ring and represents hydrogen or lower alkyl, or a pharmaceutically acceptable salt thereof, "lower" denoting that the groups or substituents so defined contain up to 7 carbon atoms.

**9.** A process according to claim 6 for the preparation of a compound of formula IIa wherein $R_1$ represents hydrogen or lower alkyl, $R_2$ represents hydrogen, $R_3$ represents hydrogen, lower alkyl, trifluoromethyl, halogen or phenyl, and $R_4$, $R_5$ and $R_6$ represent hydrogen, or a pharmaceutically acceptable salt thereof, "lower" denoting that the groups or substituents so defined contain up to 7 carbon atoms.

**10.** A process according to any one of claims 1 to 3 for the preparation of a compound of formula IIb

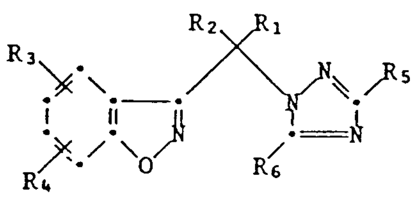

(IIb)

wherein $R_1$ and $R_2$ independently represent hydrogen, lower alkyl, aryl-lower alkyl, lower alkenyl or lower alkynyl, or $R_1$ and $R_2$ together represent lower alkylene, $R_3$ and $R_4$ independently represent hydrogen, lower alkyl, cycloalkyl, halogen, trifluoromethyl, cyano, nitro, amino, hydroxy, lower alkanoyloxy, carbocyclic aroyloxy, lower alkoxy or carbocyclic aryl, or $R_3$ and $R_4$ together, when located on adjacent carbon atoms, represent lower alkylenedioxy, and $R_5$ and $R_6$ independently represent hydrogen or lower alkyl, or a pharmaceutically acceptable salt of that compound, "lower" denoting that the groups or substituents so defined contain up to 7 carbon atoms.

**11.** A process according to claim 1 for the preparation of 5-chloro-3-[1-(1H-imidazol-1-yl)ethyl]-1,2-benzisoxazole or a pharmaceutically acceptable salt thereof.

**12.** A process according to claim 1 for the preparation of the dextrorotatory antipode of 5-chloro-3-[1-(1H-imidazol-1-yl)ethyl]-1,2-benzisoxazole or a pharmaceutically acceptable salt thereof.

## Revendications

### Revendications pour les Etats contractants suivants : AT, BE, CH, DE, FR, GB, IT, LI, LU, NL, SE

**1.** Composés de formule I

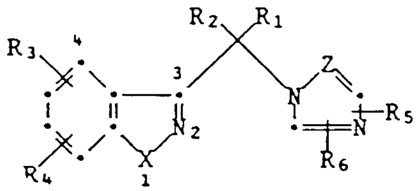

(I)

dans laquelle X représente l'oxygène ou le soufre, Z représente le groupe -CH= pour formation du groupe imidazole-1-yle ou un hétéroatome d'azote pour formation du groupe 1,2,4-triazole-1-yle, $R_1$ et $R_2$ représentent chacun, indépendamment l'un de l'autre, l'hydrogène, un groupe alkyle inférieur, alcényle inférieur, alcynyle inférieur ou aryl-alkyle inférieur, ou bien $R_1$ et $R_2$ forment ensemble un groupe alkylène inférieur, ou bien $R_1$ forme avec $R_5$, en tant que substituant de l'atome de carbone Z du groupe imidazolyle, un groupe alkylène en C2-C4, $R_3$ et $R_4$ représentent chacun, indépendamment l'un de l'autre, l'hydrogène, un groupe alkyle inférieur, cycloalkyle, un halogène, un groupe trifluorométhyle, cyano, nitro, amino, hydroxy, alcanoyloxy inférieur, aroyloxy carbocyclique, alcoxy inférieur ou aryle carbocyclique, $R_3$ et $R_4$ forment ensemble, sur des atomes de carbone voisins, un groupe alkylène-dioxy inférieur, $R_3$ et $R_4$ forment ensemble, sur des atomes de carbone voisins, un groupe 1,3-butadiénylène ou alkylène en C3-C5, $R_3$ et $R_4$ formant alors avec ces atomes de carbone voisins un groupe benzocondensé correspondant ou un groupe cycloalcényle en C5-C7 condensé, $R_5$, sur un atome de carbone cyclique, représente l'hydrogène, un groupe alkyle inférieur ou hydroxyalkyle inférieur, $R_6$, sur un atome de carbone cyclique, représente l'hydrogène ou un groupe alkyle inférieur, ou bien $R_5$ et $R_6$ forment ensemble un groupe alkylène en C3-C5 et Z représente alors un atome de carbone, $R_5$ et $R_6$ substituant ensemble cet atome de carbone et l'atome de carbone voisin, et les sels de ces composés, l'expression "inférieur" s'appliquant à des groupes ou substituants qui contiennent jusqu'à 7 atomes de carbone.

2. Composés de formule I de la revendication 1, dans laquelle X représente le soufre et Z le carbone.

3. Composés de formule I de la revendication 1, dans laquelle X représente le soufre et Z l'azote.

4. Composés selon revendication 1, répondant à la formule IIa

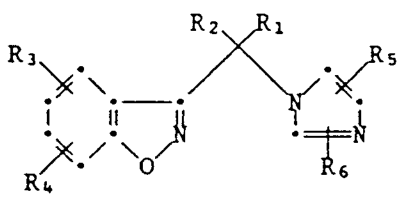

(IIa)

dans laquelle $R_1$ et $R_2$ représentent chacun, indépendamment l'un de l'autre, l'hydrogène, un groupe alkyle inférieur, aryl-alkyle inférieur, alcényle inférieur ou alcynyle inférieur ou bien $R_1$ et $R_2$ forment ensemble un groupe alkylène inférieur, $R_3$ et $R_4$ représentent chacun, indépendamment l'un de l'autre, l'hydrogène, un groupe alkyle inférieur, cycloalkyle, un halogène, un groupe trifluorométhyle, cyano, nitro, amino, hydroxy, alcanoyloxy inférieur, aroyloxy carbocyclique, alcoxy inférieur ou aryle carbocyclique ou bien $R_3$ et $R_4$ forment ensemble, sur des atomes de carbone voisins, un groupe alkylène-dioxy inférieur et $R_5$ et $R_6$ représentent chacun, indépendamment l'un de l'autre, l'hydrogène ou un groupe alkyle inférieur, et les sels acceptables pour l'usage pharmaceutique de ces composés, l'expression "inférieur" s'appliquant à des groupes ou substituants qui contiennent jusqu'à 7 atomes de carbone.

5. Composés selon revendication 4, de formule IIa dans laquelle $R_1$ représente l'hydrogène ou un groupe alkyle inférieur, $R_2$ l'hydrogène ou un groupe alkyle inférieur, $R_3$ l'hydrogène, un groupe alkyle inférieur, cycloalkyle en C5-C7, un halogène, un groupe trifluorométhyle, cyano, nitro, amino, hydroxy, alcanoyloxy inférieur, aroyloxy carbocyclique, alcoxy inférieur ou aryle carbocyclique, $R_4$ représente l'hydrogène, un groupe alkyle inférieur, alcoxy inférieur ou un halogène, ou bien $R_3$ et $R_4$ forment

ensemble et sur des atomes de carbone voisins un groupe alkylène-dioxy inférieur et $R_5$ et $R_6$ représentent chacun, indépendamment l'un de l'autre, l'hydrogène ou un groupe alkyle inférieur, et leurs sels acceptables pour l'usage pharmaceutique, l'expression "inférieur" s'appliquant à des groupes ou substituants qui contiennent jusqu'à 7 atomes de carbone.

**6.** Composés selon revendication 4, de formule IIa dans laquelle $R_1$ représente l'hydrogène ou un groupe alkyle inférieur, $R_2$ l'hydrogène ou un groupe alkyle inférieur, $R_3$ l'hydrogène, un groupe alkyle inférieur, un halogène, un groupe trifluorométhyle ou phényle, $R_4$ l'hydrogène, un groupe alkyle inférieur ou un halogène, $R_5$ est en position 4 du cycle imidazolyle et représente l'hydrogène ou un groupe alkyle inférieur, $R_6$ est en position 5 du cycle imidazolyle et représente l'hydrogène ou un groupe alkyle inférieur, et leurs sels acceptables pour l'usage pharmaceutique, l'expression "inférieur" s'appliquant à des groupes ou substituants qui contiennent jusqu'à 7 atomes de carbone.

**7.** Composés selon la revendication 4, de formule IIa dans laquelle $R_1$ représente l'hydrogène ou un groupe alkyle inférieur, $R_2$ l'hydrogène, $R_3$ l'hydrogène, un groupe alkyle inférieur, trifluorométhyle, un halogène ou un groupe phényle et $R_4$, $R_5$ et $R_6$ représentent l'hydrogène, et leurs sels acceptables pour l'usage pharmaceutique, l'expression "inférieur" s'appliquant à des groupes ou substituants qui contiennent jusqu'à 7 atomes de carbone.

**8.** Composés selon revendication 1, de formule IIb

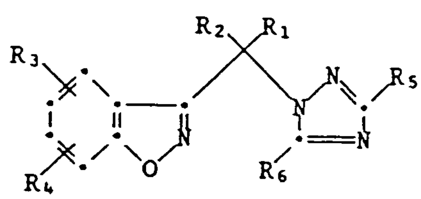

(IIb)

dans laquelle $R_1$ et $R_2$ représentent chacun, indépendamment l'un de l'autre, l'hydrogène, un groupe alkyle inférieur, aryl-alkyle inférieur, alcényle inférieur ou alcynyle inférieur, ou bien $R_1$ et $R_2$ forment ensemble un groupe alkylène inférieur, $R_3$ et $R_4$ représentent chacun, indépendamment l'un de l'autre, l'hydrogène, un groupe alkyle inférieur, cycloalkyle, un halogène, un groupe trifluorométhyle, cyano, nitro, amino, hydroxy, alcanoyloxy inférieur, aroyloxy carbocyclique, alcoxy inférieur ou aryle carbocyclique, ou bien $R_3$ et $R_4$ forment ensemble et sur des atomes de carbone voisins un groupe alkylènedioxy inférieur, et $R_5$ et $R_6$ représentent chacun, indépendamment l'un de l'autre, l'hydrogène ou un groupe alkyle inférieur, et leurs sels acceptables pour l'usage pharmaceutique, l'expression "inférieur" s'appliquant à des groupes ou substituants qui contiennent jusqu'à 7 atomes de carbone.

**9.** Le 5-chloro-3-[1-(1H-imidazole-1-yl)-éthyl]-1,2-benzoisoxazole selon revendication 1, et un sel acceptable pour l'usage pharmaceutique de ce composé.

**10.** L'antipode dextrogyre du 5-chloro-3-[1-(1H-imidazole-1-yl)-éthyl]-1,2-benzoisoxazole et un sel acceptable pour l'usage pharmaceutique de ce composé.

**11.** Compositions pharmaceutiques contenant des composés selon revendication 1 à 10, ou un sel acceptable pour l'usage pharmaceutique d'un tel composé, avec un véhicule pharmaceutique approprie.

**12.** Les composés mentionnés dans les revendications 1 à 10 pour l'utilisation dans un procédé pour le traitement thérapeutique de l'organisme humain ou animal.

**13.** Les composés mentionnés dans les revendications 1 à 10 pour l'utilisation en tant qu'agents antiépileptiques.

**14.** Procédé de préparation des composés de formule I de la revendication 1, dans laquelle tous les symboles ont les significations indiquées dans la revendication 1, et de leurs sels, caractérisé en ce

que l'on condense un composé de formule V

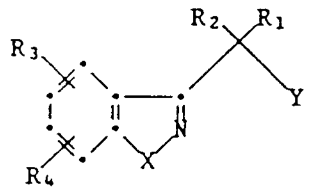

(V)

dans laquelle $R_1$ à $R_4$ et X ont les significations indiquées ci-dessus, et Y représente un groupe hydroxy estérifié réactif, avec un composé de formule VIa ou VIb.

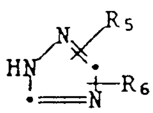

(VIa)

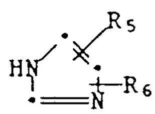

(VIb)

dans lesquelles $R_5$, sur un atome de carbone cyclique, représente l'hydrogène, un groupe alkyle inférieur ou hydroxyalkyle inférieur, de préférence à l'état protégé, et $R_6$, sur un atome de carbone cyclique, représente l'hydrogène ou un groupe alkyle inférieur, ou bien $R_5$ et $R_6$ forment ensemble un groupe alkylène en C3-C5 et Z représente alors un atome de carbone, $R_5$ et $R_6$ substituant ensemble cet atome de carbone et l'atome de carbone voisin, et si on le désire, on protège transitoirement les groupes fonctionnels réactifs dans l'exécution de l'une des opérations en question puis on isole le composé libre de formule I et/ou on convertit un composé obtenu répondant à la formule I en un autre composé de formule I et/ou on convertit un composé obtenu à l'état libre en un sel ou un sel en le composé libre ou en un autre sel et/ou on sépare un mélange d'isomères ou de racémates en les isomères ou racémates individuels et/ou on résout un racémate en les antipodes optiques, l'expression "inférieur" s'appliquant à des groupes ou substituants qui contiennent jusqu'à 7 atomes de carbone.

**15.** Procédé de préparation d'un composé de formule I de la revendication 1 dans laquelle Z représente le groupe -CH= et les autres symboles ont les significations indiquées dans la revendication 1, et de ses sels, caractérisé en ce que l'on condense un composé de formule V telle que définie dans la revendication 14 avec un composé de formule VII

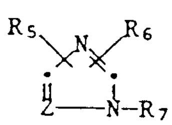

(VII)

dans laquelle $R_5$ représente l'hydrogène, un groupe alkyle inférieur ou hydroxyalkyle inférieur, $R_6$ représente l'hydrogène ou un groupe alkyle inférieur ou bien $R_5$ et $R_6$ forment ensemble un groupe alkylène en C3-C5 et Z représente alors un atome de carbone, $R_5$ et $R_6$ substituant ensemble cet atome de carbone et l'atome de carbone voisin, et $R_7$ représente un groupe protecteur usuel pour le groupe amino, puis on élimine le groupe protecteur du groupe amino, de préférence in situ, et si on le désire, on protège transitoirement les groupes fonctionnels réactifs lors de l'exécution de l'une des opérations en question puis on isole le composé libre de formule I et/ou on convertit un composé obtenu répondant à la formule I en un autre composé de formule I et/ou on convertit un composé obtenu à l'état libre en un sel ou un sel en le composé libre ou en un autre sel et/ou on sépare un mélange d'isomères ou de racémates en les isomères ou racémates individuels et/ou on résout un racémate en les antipodes optiques, l'expression "inférieur" s'appliquant à des groupes ou substituants qui contiennent jusqu'à 7 atomes de carbone.

**16.** Procédé de préparation des composés de formule I de la revendication 1 dans laquelle Z représente le groupe =CH- et $R_1$ forme avec $R_5$, en tant que substituant de l'atome de carbone du groupe imidazolyle, un groupe alkylène en C2-C4, et de leurs sels, caractérisé en ce que l'on cyclise en présence d'une base un composé de formule VIII

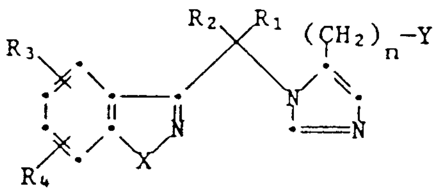

(VIII)

dans laquelle X, $R_3$ et $R_4$ ont les significations indiquées dans la revendication 1, $R_2$ a les significations indiquées dans la revendication 1 sauf un groupe alkylène inférieur en commun avec $R_1$, et Y représente un groupe hydroxy estérifié réactif, et si on le désire, on protège transitoirement des groupes fonctionnels réactifs lors de la mise en oeuvre de l'une des opérations en question puis on isole le composé libre de formule I et/ou on convertit un composé de formule I ainsi obtenu en un autre composé de formule I et/ou un composé obtenu à l'état libre en un sel ou un sel en le composé libre ou en un autre sel et/ou on sépare un mélange d'isomères ou de racémates en les isomères ou racémates individuels et/ou on résout un racémate en les antipodes optiques, l'expression "inférieur" s'appliquant à des groupes ou substituants qui contiennent jusqu'à 7 atomes de carbone.

**Revendications pour les Etats contractants suivants : ES, GR**

**1.** Procédé de préparation des composés de formule I

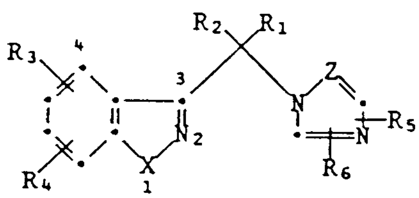

(I)

dans laquelle X représente l'oxygène ou le soufre, Z le groupe -CH= pour formation du groupe imidazole-1-yle ou un hétéroatome d'azote pour formation du groupe 1,2,4-triazole-1-yle, $R_1$ et $R_2$ représentent chacun, indépendamment l'un de l'autre, l'hydrogène, un groupe alkyle inférieur, alcényle inférieur en C3-C7, alcynyle inférieur en C3-C7 ou aryl-alkyle inférieur, ou bien $R_1$ et $R_2$ forment ensemble un groupe alkylène inférieur, ou bien $R_1$ forme avec $R_5$, en tant que substituant de l'atome de carbone Z du groupe imidazolyle, un groupe alkylène en C2-C4, $R_3$ et $R_4$ représentent chacun, indépendamment l'un de l'autre, l'hydrogène, un groupe alkyle inférieur, cycloalkyle, un halogène, un groupe trifluorométhyle, cyano, nitro, amino, hydroxy, alcanoyloxy inférieur, aroyloxy carbocyclique, alcoxy inférieur ou aryle carbocyclique, $R_3$ et $R_4$ forment ensemble et sur des atomes de carbone voisins un groupe alkylène-dioxy inférieur, $R_3$ et $R_4$ forment ensemble et sur des atomes de carbone voisins un groupe 1,3-butadiénylène ou alkylène en C3-C5, $R_3$ et $R_4$ formant alors avec ces atomes de carbone voisins un groupe benzocondensé correspondant ou un groupe cycloalcényle en C5-C7 condensé, $R_5$, sur un atome de carbone cyclique, représente l'hydrogène, un groupe alkyle inférieur ou hydroxyalkyle inférieur, $R_6$, sur un atome de carbone cyclique, représente l'hydrogène ou un groupe alkyle inférieur, ou bien $R_5$ et $R_6$ forment ensemble un groupe alkylène en C3-C5 et Z représente alors un atome de carbone, $R_5$ et $R_6$ substituant ensemble cet atome de carbone et l'atome de carbone voisin, et des sels de ces composés, caractérisé en ce que l'on condense un compose de formule V

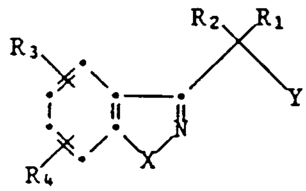

(V)

dans laquelle $R_1$ à $R_4$ et X ont les significations indiquées ci-dessus, et Y représente un groupe hydroxy estérifié réactif, avec un composé de formule VIa ou VIb

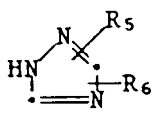

(VIa)

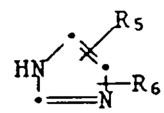

(VIb)

dans lesquelles $R_5$, sur un atome de carbone cyclique, représente l'hydrogène, un groupe alkyle inférieur ou hydroxyalkyle inférieur, de préférence à l'état protégé, et $R_6$, sur un atome de carbone cyclique, représente l'hydrogène ou un groupe alkyle inférieur, ou bien $R_5$ et $R_6$ forment ensemble un groupe alkylène en C3-C5 et Z représente alors un atome de carbone, $R_5$ et $R_6$ substituant ensemble cet atome de carbone et l'atome de carbone voisin, et si on le désire, on protège transitoirement des groupes fonctionnels réactifs dans la mise en oeuvre de l'une des opérations en question, puis on isole le composé libre de formule I et/ou on convertit un composé obtenu répondant à la formule I en un autre composé de formule I et/ou on convertit un composé obtenu à l'état libre en un sel ou un sel en le composé libre ou en un autre sel, et/ou on sépare un mélange d'isomères ou de racémates en les isomères ou racémates individuels, et/ou on résout un racémate en les antipodes optiques, l'expression "inférieur" s'appliquant à des groupes ou substituants qui contiennent jusqu'à 7 atomes de carbone.

**2.** Procédé de préparation d'un composé de formule I de la revendication 1 dans laquelle Z représente le groupe -CH= et les autres symboles ont les significations indiquées dans la revendication 1, et de ses sels, caractérisé en ce que l'on condense un composé de formule V définie dans la revendication 14 avec un composé de formule VII

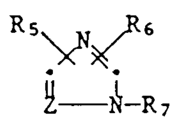

(VII),

dans laquelle $R_5$ représente l'hydrogène, un groupe alkyle inférieur ou hydroxyalkyle inférieur, $R_6$ représente l'hydrogène ou un groupe alkyle inférieur ou bien $R_5$ et $R_6$ forment ensemble un groupe alkylène en C3-C5 et Z représente alors un atome de carbone, $R_5$ et $R_6$ substituant ensemble cet atome de carbone et l'atome de carbone voisin, et $R_7$ représente un groupe protecteur usuel pour les groupes amino, puis on élimine le groupe protecteur du groupe amino, de préférence in situ, et si on le désire, on protège transitoirement les groupes fonctionnels réactifs lors de la mise en oeuvre d'une des opérations mentionnées puis on isole le composé libre de formule I et/ou on convertit un composé obtenu répondant à la formule I en un autre composé de formule I et/ou on convertit un composé obtenu à l'état libre en un sel ou un sel en le composé libre ou en un autre sel et/ou on sépare un mélange d'isomères ou de racémates en les isomères ou racémates individuels et/ou on résout un racémate en les antipodes optiques, l'expression "inférieur" s'appliquant à des groupes ou substituants qui contiennent jusqu'à 7 atomes de carbone.

**3.** Procédé de préparation des composés de formule I de la revendication 1 dans laquelle Z représente le groupe =CH- et $R_1$ forme avec $R_5$, en tant que substituant de l'atome de carbone du groupe

imidazolyle, un groupe alkylène en C2-C4, et de leurs sels, caractérisé en ce que l'on cyclise en présence d'une base un composé de formule VIII

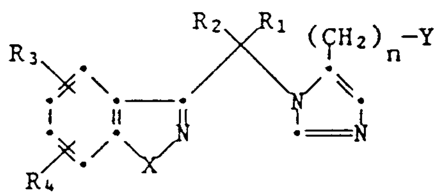

(VIII);

dans laquelle X, $R_3$ et $R_4$ ont les significations indiquées dans la revendication 1, $R_2$ a les significations indiquées dans la revendication 1 à l'exception d'un groupe alkylène inférieur en commun avec $R_1$, et Y représente un groupe hydroxy estérifié réactif, et si on le désire, on protège transitoirement des groupes fonctionnels réactifs lors de l'exécution de l'une des opérations décrites puis on isole le composé libre de formule I et/ou on convertit un composé obtenu répondant à la formule I en un autre composé de formule L et/ou on convertit un composé obtenu à l'état libre en un sel ou un sel en le composé libre ou en un autre sel et/ou on sépare un mélange d'isomères ou de racémates en les isomères ou racémates individuels et/ou on résout un racémate en les antipodes optiques, l'expression "inférieur" s'appliquant à des groupes ou substituants qui contiennent jusqu'à 7 atomes de carbone.

4. Procédé selon l'une des revendications 1, 2 ou 3, pour la préparation des composés de formule I dans laquelle X représente le soufre et Z le carbone.

5. Procédé selon l'une des revendications 1, 2 ou 3, pour la préparation des composés de formule I dans laquelle X représente le soufre et Z l'azote.

6. Procédé selon l'une des revendications 1 ou 2, pour la préparation des composés de formule IIa

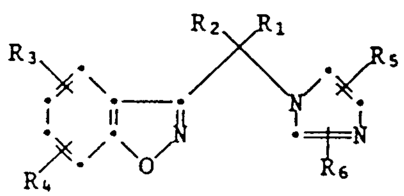

(IIa)

dans laquelle $R_1$ et $R_2$ représentent chacun, indépendamment l'un de l'autre, l'hydrogène, un groupe alkyle inférieur, aryl-alkyle inférieur, alcényle inférieur ou alcynyle inférieur, ou bien $R_1$ et $R_2$ forment ensemble un groupe alkylène inférieur, $R_3$ et $R_4$ représentent chacun, indépendamment l'un de l'autre, l'hydrogène, un groupe alkyle inférieur, cycloalkyle, un halogène, un groupe trifluorométhyle, cyano, nitro, amino, hydroxy, alcanoyloxy inférieur, aroyloxy carbocyclique, alcoxy inférieur ou aryle carbocyclique, ou bien $R_3$ et $R_4$ forment ensemble et sur des atomes de carbone voisins un groupe alkylène dioxy inférieur, et $R_5$ et $R_6$ représentent chacun, indépendamment l'un de l'autre, l'hydrogène ou un groupe alkyle inférieur, et des sels acceptables pour l'usage pharmaceutique de ces composés, l'expression "inférieur" s'appliquant à des groupes ou substituants qui contiennent jusqu'à 7 atomes de carbone.

7. Procédé selon la revendication 6 pour la préparation des composés de formule IIa dans laquelle $R_1$ représente l'hydrogène ou un groupe alkyle inférieur, $R_2$ l'hydrogène ou un groupe alkyle inférieur, $R_3$ l'hydrogène, un groupe alkyle inférieur, cycloalkyle en C5-C7, un halogène, un groupe trifluorométhyle, cyano, nitro, amino, hydroxy, alcanoyloxy inférieur, aroyloxy carbocyclique, alcoxy inférieur ou aryle carbocyclique, $R_4$ représente l'hydrogène, un groupe alkyle inférieur, alcoxy inférieur ou un halogène ou bien $R_3$ et $R_4$ forment ensemble et sur des atomes de carbone voisins un groupe alkyle inférieur, et de leurs sels acceptables pour l'usage pharmaceutique, l'expression "inférieur" s'appliquant aux groupes ou substituants qui contiennent jusqu'à 7 atomes de carbone.

**8.** Procédé selon la revendication 6 pour la préparation des composés de formule IIa dans laquelle $R_1$ représente l'hydrogène ou un groupe alkyle inférieur, $R_2$ représente l'hydrogène ou un groupe alkyle inférieur, $R_3$ représente l'hydrogène, un groupe alkyle inférieur, un halogène, un groupe trifluorométhyle ou phényle, $R_4$ représente l'hydrogène, un groupe alkyle inférieur ou un halogène, $R_5$ est en position 4 du cycle imidazolyle et représente l'hydrogène ou un groupe alkyle inférieur, $R_6$ est en position 5 du cycle imidazolyle et représente l'hydrogène ou un groupe alkyle inférieur, et de leurs sels acceptables pour l'usage pharmaceutique, l'expression "inférieur" s'appliquant aux groupes ou substituants qui contiennent jusqu'à 7 atomes de carbone.

**9.** Procédé selon la revendication 6 pour la préparation des composés de formule IIa dans laquelle $R_1$ représente l'hydrogène ou un groupe alkyle inférieur, $R_2$ l'hydrogène, $R_3$ l'hydrogène, un groupe alkyle inférieur, trifluorométhyle, un halogène ou un groupe phényle et $R_4$, $R_5$ et $R_6$ représentent l'hydrogène, et de leurs sels acceptables pour l'usage pharmaceutique, l'expression "inférieur" s'appliquant aux groupes ou substituants qui contiennent jusqu'à 7 atomes de carbone.

**10.** Procédé selon l'une des revendications 1 à 3, pour la préparation des composés de formule IIb

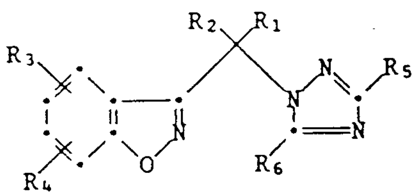

(IIb)

dans laquelle $R_1$ et $R_2$ représentent chacun, indépendamment l'un de l'autre, l'hydrogène, un groupe alkyle inférieur, aryl-alkyle inférieur, alcényle inférieur ou alcynyle inférieur, ou bien $R_1$ et $R_2$ forment ensemble un groupe alkylène inférieur, $R_3$ et $R_4$ représentent chacun, indépendamment l'un de l'autre, l'hydrogène, un groupe alkyle inférieur, cycloalkyle, un halogène, un groupe trifluorométhyle, cyano, nitro, amino, hydroxy, alcanoyloxy inférieur, aroyloxy carbocyclique, alcoxy inférieur ou aryle carbocyclique, ou bien $R_3$ et $R_4$ forment ensemble et sur des atomes de carbone voisins un groupe alkylènedioxy inférieur, et $R_5$ et $R_6$ représentent chacun, indépendamment l'un de l'autre, l'hydrogène ou un groupe alkyle inférieur, et de leurs sels acceptables pour l'usage pharmaceutique, l'expression "inférieur" s'appliquant aux groupes ou substituants qui contiennent jusqu'à 7 atomes de carbone.

**11.** Procédé selon la revendication 1 pour la préparation du 5-chloro-3-[1-(1H-imidazole-1-yl)-éthyl]-1,2-benzoisoxazole et d'un sel acceptable pour l'usage pharmaceutique de ce composé.

**12.** Procédé selon la revendication 1, pour la préparation de l'antipode dextrogyre du 5-chloro-3-[1-(1H-imidazole-1-yl)-éthyl]-1,2-benzoisoxazole et d'un sel acceptable pour l'usage pharmaceutique de ce composé.